# EUROPEAN PATENT SPECIFICATION

(11) **EP 3 082 466 B1**
(45) Date of publication and mention of the grant of the patent: **21.08.2019**
(21) Application number: 14824825.5
(22) Date of filing: 18.12.2014
(51) Int. Cl.: A23L 33/10, A23L 33/00, A61K 35/12, A61P 25/18, A61P 25/32, A61P 25/34

(54) **USE OF THYLAKOIDS TO REDUCE THE URGE FOR PALATABLE FOOD**
VERWENDUNG VON THYLAKOIDEN ZUR REDUZIERUNG DES DRANGS NACH WOHLSCHMECKENDEN NAHRUNGSMITTELN
UTILISATION DE THYLAKOÏDES POUR RÉDUIRE L'ENVIE PRESSANTE D'ALIMENT APPÉTISSANT

(30) Priority: 18.12.2013 SE 1351517
(43) Date of publication of application: 26.10.2016
(73) Proprietor: Thylabisco AB, 224 71 Lund (SE)
(72) Inventor: ALBERTSSON, Per-Åke, S-224 71 Lund (SE); ERLANSON ALBERTSSON, Charlotte, S-224 71 Lund (SE)
(74) Representative: Ström & Gulliksson AB
(86) International application number: PCT/EP2014/078377
(87) International publication number: WO 2015/091739

(56) References cited:
- WO-A1-2006/132586
- WO-A1-2010/008333
- WO-A1-2012/113918
- WO-A1-2014/040962
- US-A1- 2005 238 694
- US-A1- 2009 148 545
- US-A1- 2010 297 253
- US-A1- 2012 021 979
- KOHNKE R ET AL: "Thylakoids promote release of the satiety hormone cholecystokinin while reducing insulin in healthy humans", SCANDINAVIAN JOURNAL OF GASTROENTEROLOGY, INFORMA HEALTHCARE, UK, vol. 44, no. 6, 1 January 2009 (2009-01-01), pages 712-719, XP009159372, ISSN: 0036-5521, DOI: 10.1080/00365520902803499
- RICKARD KÖHNKE ET AL: "Thylakoids Suppress Appetite by Increasing Cholecystokinin Resulting in Lower Food Intake and Body Weight in High-fat Fed Mice", PHYTOTHERAPY RESEARCH, JOHN WILEY & SONS LTD. CHICHESTER, GB , vol. 23, no. 12 1 December 2009 (2009-12-01), pages 1778-1783, XP002676081, ISSN: 0951-418X, DOI: 10.1002/PTR.2855 Retrieved from the Internet: URL:http://onlinelibrary.wiley.com/doi/10. 1002/ptr.2855/abstract [retrieved on 2009-06-22]
- STENBLOM EVA-LENA ET AL: "Supplementation by thylakoids to a high carbohydrate meal decreases feelings of hunger, elevates CCK levels and prevents postprandial hypoglycaemia in overweight women", APPETITE, vol. 68, September 2013 (2013-09), pages 118-123, XP002736982,
- Anonymous: "Greenleaf Medical AB's Appethyl(TM) Named Finalist for Best New Ingredient NutrAward", , 19 February 2013 (2013-02-19), pages 1-2, XP055088965, Retrieved from the Internet: URL:http://www.prweb.com/releases/2013/2/p rweb10437786.htm [retrieved on 2013-11-18]

## Description

### FIELD OF INVENTION

The present invention relates to the field of compositions affecting the urge for various types of food, especially palatable food, and the reward value associated with intake of such food.

### BACKGROUND

Today, over one billion adults are overweight, that is having a body mass index (BMI) between 25-30 kg/m², and more than 300 million are obese, with BMI > 30 kg/m², worldwide. Moreover, overweight and obesity are strongly associated with hyperlipidemia, atherosclerosis and cardiovascular disease, as well as diabetes mellitus type 2.

Nowadays we have an increased availability of palatable food (e.g. sweet food, or food with high glycemic index, often rich in fat) in the western society. This may contribute to the observed global increase in body weight over the past decades. The worldwide obesity epidemic has prompted understanding of the mechanisms behind overeating and the development of efficient treatments for obesity and eating disorders. Eating beyond metabolic needs is a key event in weight gain. Palatable food, i.e. sweet and/or fat food, reinforces their intake by increasing the rewarding value and by increasing the pleasure of eating. High-risk eating episodes are those that contain palatable food, like food rich in fat and sugar. To help individuals suffering from overweight and obesity, it is therefore crucial to find ways to strengthen satiety signals and dampen hunger signals.

Furthermore, an increasing proportion of human food consumption appears to be driven by pleasure, not just by the actual need for calories (Lowe et al Physiology & Behavior Vol. 91(4), 2007, p. 432-439). It has been recognized that hunger and eating not only is subject to feedback control in relation to energy or nutrient deficits (homeostatic processes), but also from anticipated pleasure of eating (hedonic hunger). Various food stuffs have different hedonic rating. Palatable food, in particular sugar, has a high hedonic rating, i.e. provides a particular strong anticipation of rewarding. Further, hedonic hunger (the appetitive drive to eat to obtain pleasure in the absence of an energy deficit), is associated with overeating and with loss of control over eating (Witt et al International Journal of Eating Disorders Vol. 47(3), p. 273-280, 2014). Hedonic hunger favours energy-dense palatable food, rich in sugar and fat, for example snacks, pastries, desserts, baked confectionery and sweets - foods typically ingested in between meals and preferred by women (Drewnowski, A. (1997). Taste preferences and food intake. Annual review of nutrition, 17, 237-253)

Thus, also other factors than increased satiety signals and dampened homeostatic hunger signals may be relevant in treating or preventing obesity. Overweight subjects often have an increased urge for palatable food, in particular sugar (Ettinger L, Duizer L, Caldwell T: Body fat, sweetness sensitivity, and preference: determining the relationship. Can J Diet Pract Res 2012, 73(1):45-48) and fat (Blundell JE, MacDiarmid JI: Fat as a risk factor for overconsumption: satiation, satiety, and patterns of eating. J Am Diet Assoc 1997, 97(7 Suppl):S63-69).

Therefore effective treatment and prevention of obesity should preferably include strategies to suppress the urge for palatable food, especially sweet food. Of interest would further be to dampen the hedonic hunger and the wanting of such food, whereby reducing overeating and assisting in attaining a more healthy eating behavior. Further, also strategies for controlling food-reward behaviors would be of interest, as recognized by Dickson et al (J Neurosci 2012, 32(14):4812-4820). However, today no such strategies are available.

### SUMMARY

The present invention seeks to mitigate, alleviate, circumvent or eliminate at least one, such as one or more, of the above-identified deficiencies in the art.

Accordingly use of isolated thylakoids to reduce the urge for palatable food, in order to decrease hedonic hunger, in man is provided according to one aspect of the invention.

Such use of isolated thylakoids will include intake of isolated thylakoids to provide the effect. Typically the intake will provide increased release of GLP-1 (glucagon-like peptide-1), whereby the rewarding effect of palatable food, affecting the reward center in the brain, is decreased.

Further advantageous features of the invention are defined in the dependent claims. In addition, advantageous features of the invention are elaborated in embodiments disclosed herein.

### BRIEF DESCRIPTION OF DRAWINGS

These and other aspects, features and advantages of which the invention is capable of will be apparent and elucidated from the following description of embodiments of the present invention, reference being made to the accompanying drawings, in which
Figs. 1 are graphs showing the hunger sensation (A), urge for sweet food - candy (B), salt and fat food also comprising starch - potato chips (C) and sweet and fat food - chocolate (D), respectively, with and without preceding administration of thylakoids in human; and
Fig 2 is a graph showing the release of glucagon-like peptide 1 (GLP-1) during a breakfast containing sweet items with and without thylakoids.
Figs. 3A-C are graphs showing the hunger sensation experience during dietary intervention with and without thylakoids.
Figs. 4A-C are graphs showing the urge for chocolate during dietary intervention with and without thylakoids.
Figs. 5A-C are graphs showing the urge for carbohydrate snacks during dietary intervention with and without thylakoids.
Figs. 6A-D are graphs showing hunger and satiety following intake of thylakoids or placebo.
Figs. 7A-H are graphs showing VAS-ratings of wanting salty, sweet, sweet-and-fat snacks and all snacks combined, comparing treatment (thylakoids) and placebo conditions (control).
Fig. 8 shows the caloric intake from the afternoon snack buffet.
Fig. 9 shows the liking for specific food products after consumption, with and without treatment with thylakoids.
Figs. 10A-B show the correlation between treatment effect of thylakoids on wanting palatable food and emotional eating behaviour scores

### DETAILED DESCRIPTION

Several embodiments of the present invention will be described in more detail below with reference to the accompanying drawings in order for those skilled in the art to be able to carry out the invention.

The embodiments do not limit the invention, but the invention is only limited by the appended patent claims. Furthermore, the terminology used in the detailed description of the particular embodiments illustrated in the accompanying drawings is not intended to be limiting of the invention.

The present invention is based on the use of thylakoids in man, wherein the use includes intake of isolated thylakoids.

Thylakoids are membrane-bound compartments present inside chloroplasts of plants and inside cyanobacteria. The thylakoid is the site of the light-dependent reactions of photosynthesis. In the light-independent reactions of photosynthesis (i.e. the Calvin-Benson Cycle), which requires NADPH formed in light-dependent reactions of photosynthesis, carbon dioxide is converted to three-carbon sugars, which later are combined to form sucrose and starch. This process is often denoted carbon fixation. Thylakoids consist of a thylakoid membrane surrounding a thylakoid lumen. In the chloroplast, thylakoids are commonly grouped as stacks of disks being refereed to as grana.

Thylakoids may be extracted from the chloroplast of green leaves and from cyanobacteria. Their content includes proteins, lipids and pigments (e.g. chlorophyll and carotenoids). The general principles of isolating and enriching thylakoids have been described elsewhere (cf. PCT/SE2006/000676, WO/SE2009/000327, and Emek, SC, Szilagyi A, Akerlund H E, et al. (2010) A large scale method for preparation of plant thylakoids for use in body weight regulation Prepar biochem & biotech 40 (1), 13-27). In short, the cells of green leaves are broken mechanically. The thylakoids are then separated from the cell walls and other large cell fragments by filtration. Thylakoids are then collected from the filtrate by precipitation at pH 4.7 and washed in water by repeated centrifugations.

Isolated thylakoids have previously been found to increase satiety, decrease homeostatic hunger signals and promote weight loss in several studies, both in human and animal subjects (cf. WO 2006/132586 and WO 2010/008333 among others). Thylakoids, or parts thereof, may be included in foodstuff in many different ways since they are themselves food. They may also be included in different types of pharmaceutical formula as previously described in WO 2006/7132586.

The current belief in the art (cf. Stenblom et al Supplementation by thylakoids to a high carbohydrate meal decreases feelings of hunger, elevates CCK levels and prevents postprandial hypoglycaemia in overweight women Appetite 68 (2013) 118-123) is that isolated thylakoids retard lipolysis and provides decreased homeostatic hunger (i.e. the urge to eat when fasting). Intake of isolated thylakoids has been found to affect homeostatic hunger signals by increasing cholecystokinin (CCK) levels (satiety) and decreasing ghrelin levels (hunger).

Although green leaves naturally comprise thylakoids, intake of fresh green leaves does not provide the same effect as intake of isolated thylakoids, or parts thereof. Plant cells comprising thylakoids are surrounded by very thick and tough cell wall. Although we chew a lot when eating vegetables, not many cells are broken. In order to dissolve the cell walls, special enzymes such as cellulase and pectinas etc. are required. However, these enzymes are not present in the stomach or in the small intestine of humans. Unless extracted from the greens leaves, the thylakoids may not exert any effect in the small intestine before reaching the large intestine, wherein the cell walls may be broken down by bacteria.

The effect on lipolytic activity of isolated thylakoids, as well as on insulin sensitivity and uptake of various molecules, has been reported in the art (cf. e.g. WO 2006/7132586, WO 2010/008333, and WO 2012/113918). Further, the effect of intact thylakoids as radical scavengers has also been reported in the art (cf. WO 2003/004042). However, further effects of thylakoids still remain to be discovered.

As explained above, an important aspect of obesity is the tendency of obese individuals not only to overeat but also to preferentially choose palatable food, e.g. food with high glycemic index (i.e. 60 or above) often with high fat content, as well as sweet tasting food comprising sweeteners or sugars, and sweet and fat food, over healthy food. Fast-food, e.g. pizza, hamburgers, French fries, soft drinks etc., are typical examples of palatable food increasing the risk for over-eating. The preferential for palatable food is linked to the rewarding value of the food; over-eating may thus be driven by the wish to obtain pleasure.

The palatability of available food in today's society undermines normal satiety signals, motivating energy intake independent of energy need (cf. Erlanson-Albertsson C: How palatable food disrupts appetite regulation. Basic Clin Pharmacol Toxicol 2005, 97(2):61-73). Further, both sweet and fat foods mobilize opioids and dopamine within the reward system, establishing pathways for urge for such food, and even craving of, such food (cf. Kelley AE, Bakshi VP, Haber SN, Steininger TL, Will MJ, Zhang M: Opioid modulation of taste hedonics within the ventral striatum. Physiol Behav 2002, 76(3):365-377.)

Thus, reducing the urge for palatable food is extremely important in order for a weight reduction program to be successful. Further, not only obese subject would have use of a composition providing reduced urge for palatable food, but also normal weight subjects. Reducing the hedonic hunger, may facilitate in attaining a more healthy eating behavior. Similar, decreasing the rewarding effect of palatable food may also facilitate in attaining a more healthy eating behavior. Attenuating the hedonic drive in those who experience increased cravings for palatable food is a way of avoiding overeating and weight gaining.

Intake of food having high glycemic index or tasting sweet, as well as food having a high fat, content is associated with increased risk for obesity, cardiovascular diseases, including coronary diseases, and diabetes, as demonstrated in several studies (cf. Johnson RK, Appel LJ, Brands M, Howard BV, Lefevre M, Lustig RH, Sacks F, Steffen LM, Wylie-Rosett J: Dietary sugars intake and cardiovascular health: a scientific statement from the American Heart Association. Circulation 2009, 120(11):1011-1020 and Johnson RJ, Segal MS, Sautin Y, Nakagawa T, Feig DI, Kang DH, Gersch MS, Benner S, Sanchez-Lozada LG: Potential role of sugar (fructose) in the epidemic of hypertension, obesity and the metabolic syndrome, diabetes, kidney disease, and cardiovascular disease. Am J Clin Nutr 2007, 86(4):899-906. In particular sugars that contain fructose may predispose to such disease.

An important factor for the preference for palatable food is the reward value linked to it. Eating palatable food results in release of reward factors having an important role in over-eating. Could the effect of these reward factors be reduced, the preference for palatable food and also over eating would be reduced.

Further, not only food, but also other stimulants, e.g. alcohol and nicotine, are driven by the same reward factors. Since people trying to stop smoking often gain weight it would be of specific interest not only to reduce the reward value of palatable food, but also of nicotine. Similar to intake of palatable food, smoking is associated with an increased risk for cardiovascular diseases.

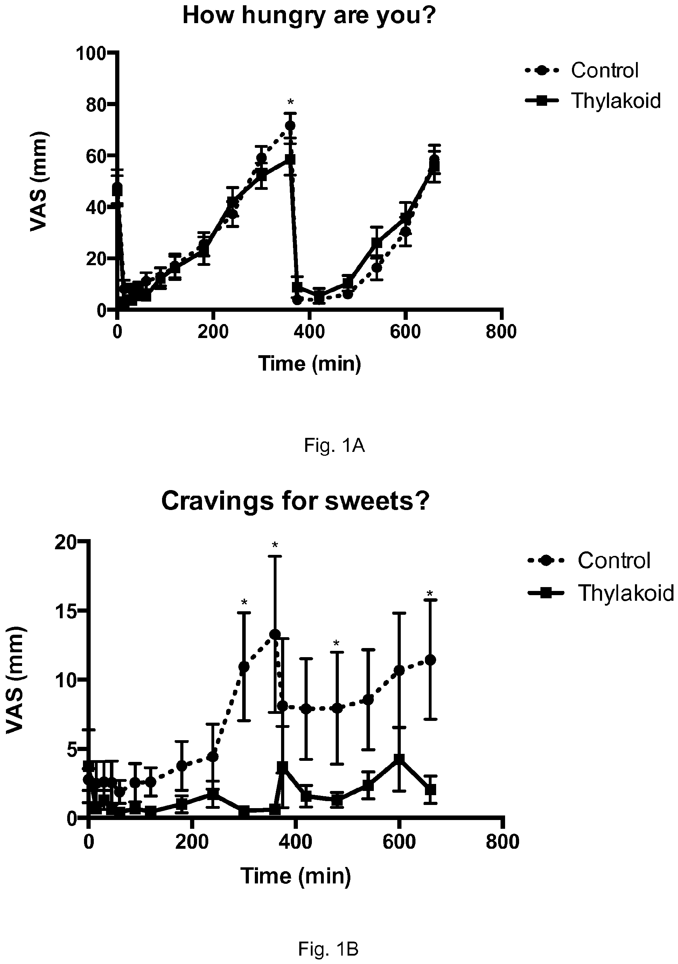

The present inventors have surprisingly revealed that intake of isolated thylakoids not only affects the lipolytic activity and thereby the appetite (homeostatic hunger - Fig. 1A), but also, and importantly, reduces the urge for palatable food (hedonic hunger - Figs. 1B-D among others). Interestingly, the finding is not related to the suppression of appetite, as the effect is being present also in hungry individuals (cf. Fig. 1A vs. Figs. 1B-D). In other words, the reduced urge for palatable food is observed not only immediately after eating but also in the late postprandial phase for at least six hours when hunger is present. However, hungry individuals having been administered a composition comprising isolated thylakoids were found to be much less prone to urge for palatable food, such as candy, chocolate and potato chips. The effect remains even after a second meal (lunch) without any thylakoids (cf. Figs. 1B-D), even though the homeostatic hunger doesn't differ (cf. Fig. 1A).

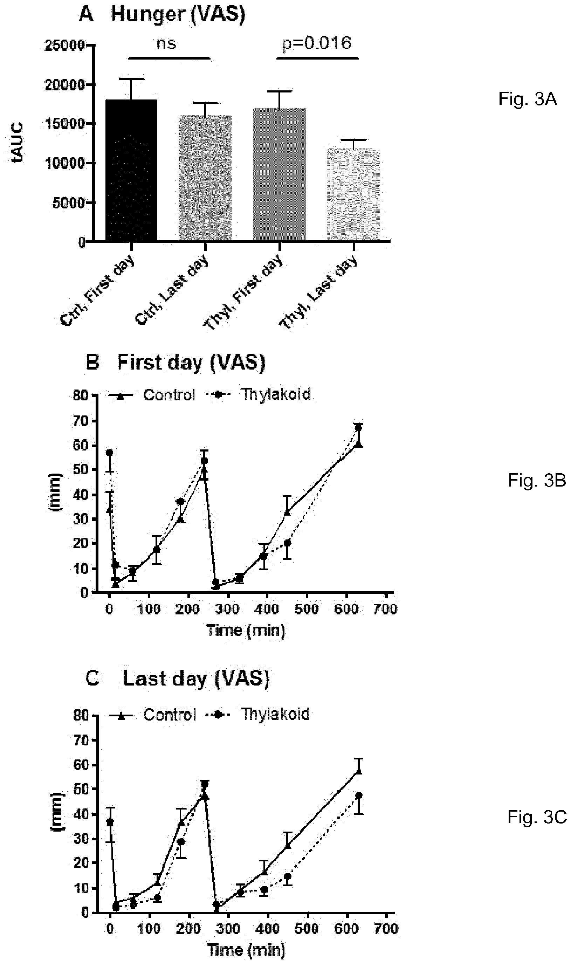

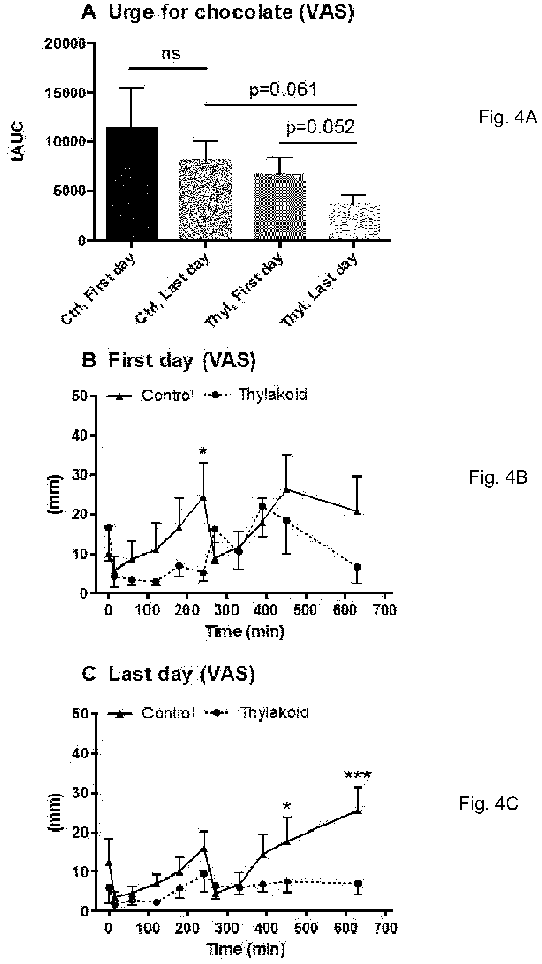

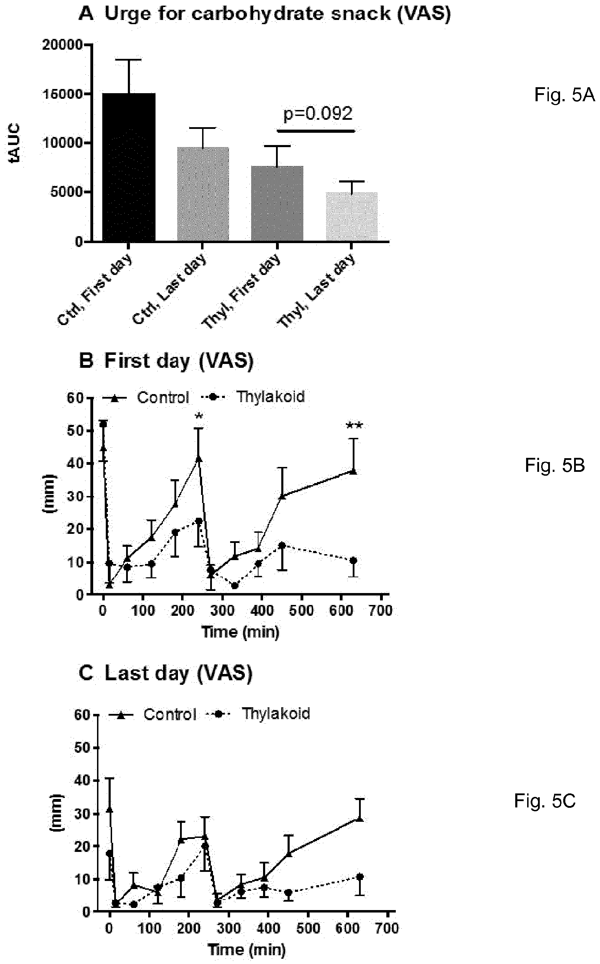

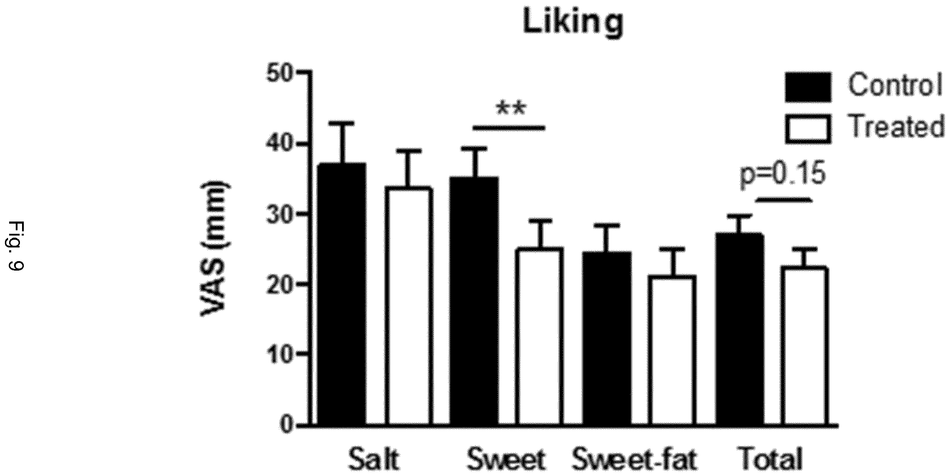

Also in subjects on dietary intervention, in which the effect on homeostatic hunger is less pronounced (cf. Figs. 3A-C), reduced hedonic hunger is attained with administration of isolated thylakoids (cf. Figs 4 and 5). Further, administration of isolated thylakoids may even result in decreased liking of sweet (cf. Fig. 9)

Without being bond to any theory, the effect of reducing the urge for palatable food is believed to be related to a previously unknown effect of increasing the release of a hormone named glucagon-like peptide-1 (GLP-1), being a hormone produced in the distal intestine by specific L-cells (Holst JJ: The physiology of glucagon-like peptide 1. Physiol Rev 2007, 87(4):1409-1439)). As shown herein below, oral intake of isolated thylakoids increases the release of GLP-1.

Interestingly, this hormone is not only involved in blood sugar regulation, but has also been shown to decease the rewarding value of food (Dickson SL, Shirazi RH, Hansson C, Bergquist F, Nissbrandt H, Skibicka KP: The glucagon-like peptide 1 (GLP-1) analogue, exendin-4, decreases the rewarding value of food: a new role for mesolimbic GLP-1 receptors. J Neurosci 2012, 32(14):4812-4820). It may thus be that the effect of reducing the urge for palatable food is linked to an increased release of GLP-1. However, also other factors yet to be revealed may be important for the new findings disclosed herein.

The finding is further interesting, as the release of GLP-1 not only affects the reward value of food but also of other stimulants including alcohol (Egecioglu E, Steensland P, Fredriksson I, Feltmann K, Engel JA, Jerlhag E: The glucagon-like peptide 1 analogue Exendin-4 attenuates alcohol mediated behaviors in rodents. Psychoneuroendocrinology 2013, 38(8):1259-1270; and Shirazi RH, Dickson SL, Skibicka KP: Gut peptide GLP-1 and its analogue, Exendin-4, decrease alcohol intake and reward. PLoS One 2013, 8(4):e61965). It is believed that the mechanism is related to suppression of dopamine in the reward centers of the brain.

Further, also the need for cigarettes and other tobacco products is related to nicotine releasing dopamine in the reward centers of the brain. The stimulating action of nicotine is attenuated by the release of GLP-1, as have been demonstrated by Egecioglu et al (cf. "The Glucagon-Like Peptide 1 Analogue Exendin-4 Attenuates the Nicotine-Induced Locomotor Stimulation, Accumbal Dopamine Release, Conditioned Place Preference as well as the Expression of Locomotor Sensitization in Mice", PloS One 2013 18:8 e 77 284.

It has unexpectedly been found that smokers that take isolated thylakoids actually experience less need to smoke (interpreted as decreased need for nicotine), whereby they also smoke a lower number of cigarettes per day. This may be an effect of an increased release of GLP-1 by thylakoids. As smokers trying to quit smoking often gain weight, the previously known effect of suppressing appetite provided by intake of isolated thylakoids provides an additional advantage when isolated thylakoids are taken by individuals trying to quit smoking.

In contrast to the previous uses of thylakoids, relying on local effects exerted by thylakoids in the intestines, the present use is believed to relate to central effects exerted by thylakoids affecting the reward center of the brain. Further, the effect is also distinct from the increased levels of CCK//decreased levels of ghrelin previously reported.

An embodiment of the present invention therefore relates to the use of isolated thylakoids to suppress, lower, decrease, reduce, lessen, and/or ease the urge for palatable food, in order to decrease hedonic hunger, in man. Such use may be non-therapeutic. Further, such use may be therapeutic. The use includes intake of isolated thylakoids. While the person taking isolated thylakoids will be less prone to select palatable food over more healthy alternatives, the person will not all the suddenly dislike palatable food. The reduced urge for palatable food will assist in treating and preventing of obesity, as persons taking isolated thylakoids will be less prone to overeat. Further, the reduced urge for palatable food may assist in maintaining the weight after successfully having taken part in a weight loss program.

Examples of palatable food include sweets, pastries, e.g. doughnuts, croissants, shortbread cookies and wafers, candy, potato chips, French fries, soft drinks, e.g. Coca Cola, Fanta, Sprite, Pepsi Cola, and energy drinks (e.g. Red Bull), chocolate, e.g. milk chocolate and chocolate bars, fast food, e.g. pizza and hamburgers, and ice-cream. Further, palatable food typically may have one or several of the following characteristics:
- a glycemic index of at least 60, such at least 70;
- high sugar content of at least 5 wt% for drinks, and at least 20 wt%, such as at least 50 wt%, for other foodstuff;
- comprising a sweetener, such as sweetener selected from the group consisting of: stevia, aspartame, sucralose, neotame, acesulfame potassium, cyclamate, alitame, mogrosides, dulcin, glucin, neohesperidin dihydrochalcone, saccharin, brazzein, curculin, glycyrrhizin, mabinlin, monellin, pentadin, and thaumatin; especially a sweetener selected from the group consisting of stevia, aspartame, sucralose, neotame, acesulfame potassium, and saccharin;

- a high fat content of at least 20 wt% and high content of sugar of at least 20 wt%;
- a high fat content of at least 20 wt% and a glycemic index of at least 50; and.
- a high-fat content of at least 20 wt% and salt content (NaCl) of at least 1.0 wt%.

Typically, palatable food has a high glycemic index and/or tastes sweet. Also sweet food, not having a high GI, e.g. food comprising fructose or a sweetener may trigger a reward effect and hence be involved in over eating. As known to the skilled person intake of diet soft drinks also provides a rewarding effect, which indirectly contributes to over-eating. Diet drinks do cause a release of dopamine, demonstrating that they also have a rewarding effect that can lead to overeating. Further, also food having a moderate GI of at least 50 and high fat content, such as French fries, will trigger a reward effect and may hence be involved in over eating. Even salty snacks with high fat content, such as salted nuts, trigger a reward effect.

According to an embodiment, sugars present in the palatable food in amounts herein specified above are selected from the group consisting of the monosaccharides glucose (also known as dextrose), fructose and galactose and the disaccharides sucrose, maltose and lactose, and mixtures thereof.

Another embodiment relates to the use of isolated thylakoids, or parts thereof, to decrease the rewarding effect of food, such as palatable food. Such use may be non-therapeutic. Further, such use may be therapeutic. Examples of palatable food have been provided herein above.

While the present invention has been described as relating to the use of isolated thylakoids to suppress, lower, decrease, reduce, lessen, and/or ease the urge for palatable food, it, according to another embodiment, relates to a method of suppressing, lowering, decreasing, reducing, lessening, and/or easing the urge for palatable food. Such a method comprises the step of administering isolated thylakoids, or parts thereof, to a subject, e.g. a human being, whose urge for palatable food is to be affected.

As already described, intake of thylakoids, or parts thereof, has also been shown to reduce the need or urge to smoke in man; probably by affecting the reward center of the brain. Not only palatable food and nicotine is affecting the reward center of the brain, but also other stimulants, such as alcohol, i.e. ethanol.

As explained herein above, the effect of intake of isolated thylakoids, or parts thereof, on the urge for various stimuli, including palatable food, nicotine and alcohol, is believed to be linked to an increase in the release of GLP-1 (glucagon-like peptide-1). Thus, a further embodiment relates to the use of isolated thylakoids, or parts thereof, to release GLP-1 (glucagon-like peptide-1) in man and to a method of releasing GLP-1 (glucagon-like peptide-1) in man including administration of isolated thylakoids, or parts thereof. Such use may be non-therapeutic. Further, may such use be therapeutic. Furthermore, GLP-1 is known to decrease the rewarding effects of various stimuli, including palatable food, nicotine and alcohol in the reward center in the brain. Isolated thylakoids, or parts thereof may thus also be used to decrease the rewarding effect of stimulants affecting the reward center in the brain. Similarly, isolated thylakoids, or parts thereof may be used in a method of decreasing the rewarding effect of stimulants affecting the reward center in the brain.

Thylakoids may be isolated from plants as well as from cyanobacteria. Typically, the thylakoids are enriched from green leaves or green algae. The thylakoids may origin from leaves of any photosynthesizing plants, such as clover, rape, sugar beet, dandelion, Arabidopsis thaliana, maize, tobacco, sun flower, Chenopodium, Atriplex, spinach, mangold, quinoa, kale, sugar beet and grasses. Preferably, the thylakoids are isolated and enriched from spinach. The isolated thylakoids among other parts of the thylakoid, comprises thylakoid membranes, or parts thereof. Methods for isolating thylakoids have been discussed herein above.

The thylakoids may be isolated and concentrated in a manner such that the chlorophyll content of the isolated thylakoids is from about 8 mg to 150 mg, such as 10-100 mg, chlorophyll per g isolated thylakoids. In some embodiments, the isolated thylakoids have a chlorophyll a/b ratio of from about 2.0 to 4.0, preferably about 2.6 to 3.4, and most preferably about 2.9 to 3.4. Further, the protein:chlorophyll ratio in the isolated thylakoids may be from about 3.0 to about 10.0, preferably about 2.0 to 5.0, 3.0 to 5.0, or 6.0 to 8.0.

Typically, the thylakoids are administered orally to provide their effect. While they may be administered directly, they typically are administered as an integral part of a composition, such as nutraceutical composition, e.g. a foodstuff or a dietary supplement, or pharmaceutical composition, e.g. a medicament.

If administered as medicament, the medicament, except for the isolated thylakoids, also comprises at least one pharmaceutical acceptable excipient, such as a carrier, a diluent, and/or a stabilizer. In this context, "pharmaceutically acceptable" relates to an excipient that, at the dosage and concentration employed, does not cause any unwanted effects in the subject to whom it is administered. Such pharmaceutically acceptable excipients are well-known in the art. According to an embodiment a medicament as disclosed herein may also comprise other pharmaceutically acceptable excipients, such as preservatives, antioxidants, colouring agents and the like.

According to another embodiment, the isolated thylakoids are added as food additive to a foodstuff, to provide a food stuff having the ability to affect the reward center in the brain, as described herein. Similar to the isolated thylakoids such food stuff finds none-therapeutic use.

According to yet another embodiment, the isolated thylakoids are added as an ingredient to a dietary supplement, to provide a dietary supplement having the ability to affect the reward center in the brain, as described herein. Similar to the isolated thylakoids, such a dietary supplement may find therapeutic as well as none-therapeutic use. The dietary supplement may for example be a fruit juice shoot, e.g. a blueberry shot, or a vegetable soup or shoot, e.g. potato soup, broccoli soup, potato soup, or tomato soup, in which the isolated thylakoids, or parts thereof, are added. The ingredients in the dietary supplement are such that contain antioxidants, vitamins, fibers which give further usefulness as to health.

In embodiments wherein the isolated thylakoids are administered as an integral part of a composition, the content of thylakoids in the composition may correspond to a chlorophyll content of 1 mg to 100 mg chlorophyll per g composition (dry weight). Preferably, the composition comprises from about 8 to about 80 mg chlorophyll per gram (dry weight), preferably from about 10 to about 50 mg, such as about 10 mg to 30 mg, 20 mg to 40 mg, or 30 mg to 40 mg chlorophyll content per gram (dry weight). Further, the carbohydrate content of the composition may be from about 5 to about 50 g carbohydrate content per 100 g of the composition (dry weight), preferably about 10 g to 40 g per 100 g, such as 15 g to 30 g, 20 g to 30 g, or 30 g to 40 g per 100 g (dry weight). Also, the protein content of the composition may be from about 10 to about 60 g protein per 100 g of the composition (dry weight), preferably about 10 g to 40 g per 100 g, preferably 15 g to 30 g, 20 g to 30 g, or 30 g to 40 g per 100 g (dry weight).

According to an embodiment, the composition is an oral pharmaceutical or nutraceutical composition comprising a physiologically tolerable oil-in-water emulsion comprising thylakoids. In such an emulsion, the amount of thylakoids may be 20 to 30 wt.%, such as about 25 wt.%. Further, the oil-content may be 20 to 30 wt.%, such as about 25 wt.%. The oil used may be vegetable oils of food grade, such as edible rape seed oil (also known as Canola oil), olive oil, sunflower oil etc.

As the composition may be administered in various forms, the daily dose of thylakoids to be administered may vary. As guiding principle, the amount of the composition to be administered may be selected in manner such that the dosage correlates to a daily dose of 2.4 mg to 240 mg thylakoids per kg body mass of the mammal.

Further, the present inventors have recently and surprisingly discovered (cf. PCT/EP2013/068656) that thylakoids regulate gut microbiota and hence may function as a prebiotic agent. According to an embodiment, a composition as disclosed herein therefore further comprises probiotic bacteria. The probiotic bacteria to be included in the composition may be *Bifidobacterium,* e.g. *Bifidobacterium infantis,* or *Lactobacillus,* e.g. *Lactobacillus plantarum.* Further, also prebiotic agents, such as trans-galactooligosaccharide, inulin, fructooligosaccharide (FOS), lactulose and mannan Oligosaccharides (MOS), may be included in the composition.

Without further elaboration, it is believed that one skilled in the art may, using the preceding description, utilize the present invention to its fullest extent. The above preferred specific embodiments are, therefore, to be construed as merely illustrative and not limitative to the disclosure in any way whatsoever.

Although the present invention has been described above with reference to (a) specific embodiment(s), it is not intended to be limited to the specific form set forth herein. Rather, the invention is limited only by the accompanying claims and, other embodiments than the specific above are equally possible within the scope of these appended claims, e.g. different than those described above.

In the claims, the term "comprises/comprising" does not exclude the presence of other elements or steps. Additionally, although individual features may be included in different claims, these may possibly advantageously be combined, and the inclusion in different claims does not imply that a combination of features is not feasible and/or advantageous.

In addition, singular references do not exclude a plurality. The terms "a", "an", "first", "second" etc do not preclude a plurality.

### EXPERIMENTAL

The following examples are mere examples and should by no mean be interpreted to limit the scope of the invention. Rather, the invention is limited only by the accompanying claims.

### Example 1 - decreased urge for palatable food

### Present study:

38 women were requited through posters and advertisements in the local community (Lund, Sweden). The criteria were women aged 40-65 years, BMI between 25-33, non-smoking and healthy; the exclusion criteria included diabetes, food allergies, intestinal bowel syndrome, food intolerance and recent use of antibiotics. The subjects were not vegetarian and had not followed any diet for the last three months. Baseline characteristics of the 38 included women can be seen in table 1. Two test groups were created through randomized selection. The Ethics Committee in Lund, Sweden approved the study.

**Table 1 - Baseline characteristics of the 38 women anticipating the meal intervention study.**

| | **Thylakoid (n = 19)** | **Control (n = 19)** |
|---|---|---|
| **Age (years)** | 50.2 ± 6.9 | 54.2 ± 6.9 |
| **Body weight (kg)** | 79.9 ± 10.8 | 80.2 ± 8.2 |
| **BMI (kg/m2)** | 28.9 ± 2.2 | 28.6 ± 2.3 |
| **FFM (kg)** | 47.6 ± 4.9 | 47.6 ± 3.2 |
| **Body fat (kg)** | 32.3 ± 6.8 | 32.6 ± 6.2 |
| **Body fat (%)** | 40.1 ± 4.0 | 40.4 ± 4.1 |

| | | |
|---|---|---|
| *BMI* = *Body Mass Index, defined as the relation between body mass (kg) and body length (meters) in square* *FFM* = *Fat Free Mass; includes bones, muscles and organs.* | | |

Subjects arrived fasting (i.e. no food or drink intake over night) to the clinic at 7 am the morning of the study. A venous catheter was mounted, for repetitive blood sampling throughout the day. The subjects were given an iso-caloric breakfast (table 2) of 660 kcal (60% carbohydrates, 28% fat, and 12% protein).

**Table 2 - The caloric content in each ingredient of the breakfast (first meal) specified as kcal**

| **Breakfast ingredients** | **Amount** | **Caloric content** |
|---|---|---|
| Vanilla yoghurt, 2.5% fat | 150 g | 121,5 kcal |
| Tropical musli (F-musli, AXA Lantmännen, Järna, Sweden) | 1 dl = 45 g | 185 kcal |
| White bread (Skogaholmslimpa, Pågen, Malmö, Sweden) | 1 piece = 40 g | 98 kcal |
| Butter (Bregott, Arla Foods, Stockholm, Sweden) | 1 tsp | 33 kcal |
| Cheese, 28% fat (Gouda, Friendship, Germany) | 2 pieces = 20 g | 77 kcal |
| Red bell pepper | 2 pieces = 20 g | 6 kcal |
| Orangejuice (Bravo, Skånemejerier, Lunnarp, Sweden) | 2 dl | 86 kcal |
| Coffee/tea (with 1 tbsp milk 0.5% fat if preferred) | 1 cup | ∼10 kcal |
| Blueberry shot (+/- thylakoid supplement) | 1 shot | 45 kcal |
| | | **660 kcal** |

A 50 g blueberry shot with or without supplementation of 5 g thylakoids were taken approximately five minutes before their breakfast. The subjects were told to eat all given food within 15 minutes. No drinking or eating was permitted between meals. A second meal consisting of a pizza (Grandiosa Gudfadern, Procordia AB, Eslöv, Sweden), water and coffee/tea were given to subjects after 6 hours with the instruction to eat and drink until satisfied. The second meal was served in a cubicle for each subject one by one. A third meal, dinner, consisting of a salmon "*pytt i panna*" (Laxpytt, Findus AB, Bjuv, Sweden) was given to the participants to consume a freely chosen amount of in the evening at home.

Blood samples were taken just before breakfast (time point: 0 minute) and thereafter at 15, 30, 45, 60, 90, 120, 180, 240, 300 and at 360 minutes. The lunch was given just after the blood sampling at time point 360 minute. Analysis was carried out for a number of components including GLP-1.

Questionnaires (designed as Visual Analogue Scale, VAS) regarding feelings of hunger, fullness and urge for different foods was answered at the same time points as blood samples were taken throughout the day.

The questionnaire was also answered between the second and third meal at time points 60, 120, 180 and 240 minutes after lunch.

The thylakoid powder used in the present meal study was produced as Appethyl, by Future-ceuticals, Chicago, IL, USA. 5 gram of the powder were mixed with 50 g blueberry juice (Ekströms Blåbärssoppa Orginal, Procordia AB, Eslöv, Sweden) and 2 g rapeseed oil (Zeta rapsolja, DILUCA & DI LUCA AB, Stockholm, Sweden). The control was consisting of 50 g blueberry juice and 2 g rapeseed oil.

The plasma GLP-1 analys was made with an enzyme linked immunosorbent assay (ELISA) kit (Cat. # EGLP-35K; Millipore Corp. Billerica, MA, USA). The kit analyses only the biologically active forms of GLP-1: GLP-1 (7-36 amide) and GLP1 (7-37 amide) in human plasma. A fluorescence plate reader (FLUOstar OPTIMA 413-101; BMG LABTECH GmbH, Ortenberg, Germany) with an excitation wavelength of 355 nm and emission wavelength of 460 nm were used for reading the samples. The inter-assay coefficient of variation (CV) is between 1-13 % and intra-assay between 6-9 %, accordning to the manufactures data.

A visual Analogue Scale (VAS) questionnaire was used to estimate the subjects feeling of hunger, fullness and cravings for sugar, sugar/fat and salt/fat. The subjects were given questions on a paper and were told to estimate their feelings and thoughts by writing a vertical line on a horizontal linear scale. The scale was 100 mm in length and was anchored by sensory descriptions (table 3).

**Table 3. The questions in the VAS questionnaire with anchored phrases and scores.**

| **Questions** | **Anchor phrases** | |
|---|---|---|
| | Score 0 | Score 100 |
| How hungry are you feeling right now? | Not at all hungry | Extremely hungry |
| How full are you right now? | Not at all full | Extremely full |
| How much are you thinking of food right now? | Not at all | Extremely |
| How much do you urge for a sandwich [image picturing sandwich with cheese and red bell pepper]? | Not at all | Extremely |
| How much do you urge for chips [image picturing salty chips]? | Not at all | Extremely |
| How much do you urge for chocolate [image picturing milk chocolate] | Not at all | Extremely |
| How much do you urge for candy [image picturing pic & mix candy]? | Not at all | Extremely |

The diet regime (i.e. intake of 5 g thylakoids per day) continued for three months.

### Results

A significant reduction in the urge for palatable food was observed in conjunction with intake of thylakoids (cf. Figs. 1B-D). The decreased urge occurred for sweets (goodies), for fat and sweet (chocolate), and also for fat and salt (chips). It is thus concluded that thylakoids have the ability to suppress urge or desire for sweet, salt and fat. This suppression of desire lasts the whole day following consumption of a thylakoid shot in the morning. Furthermore, the liking of palatable food decreased further after 3 months of thylakoid treatment.

Importantly, the effect on hunger was much less pronounced (cf. Fig. 1A). Despite being hungry, the women had significantly less desire for palatable food, such as candy, chocolate or potato chips. The effect underlying the present invention is thus apparently not related to the previous findings that intake of enriched, isolated thylakoids may suppress appetite, due to decreased lipolytic activity (cf.
WO 2006/132586), and delay uptake of molecules, such as glucose (cf.
WO 2012/113918).

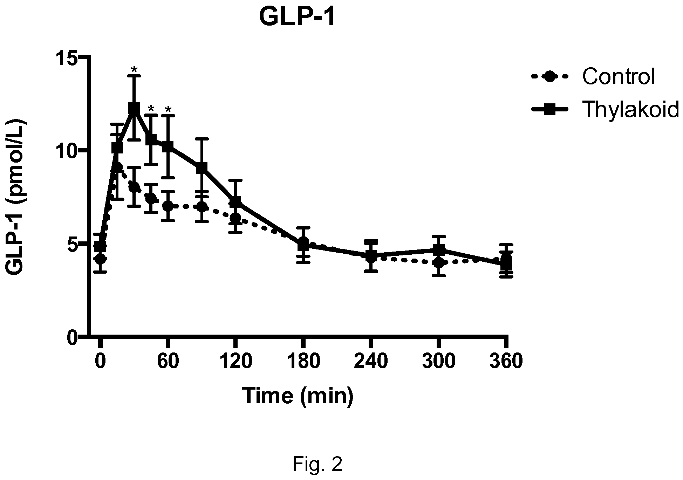

Without being bond to any theory, it seems that the mechanism probably is related to the release of glucagon-like peptide (GLP-1), which was significantly released by thylakoids compared to the control (Fig. 2). The thylakoids thus cause endogenous release of a hormone that regulates reward-related eating behavior in a direction that is helpful to prevent and treat obesity. Especially, subjects having taken thylakoids will be more prompted to choose a healthy meal as the urge for palatable food is significantly reduced. Further, the risk for over-eating is reduced. This will lead to a decreased risk for type-2-diabetes and cardiovascular disease associated with obesity and insulin resistance.

### Example 2 - decreased need for nicotine (not within the scope of the invention)

As well known, smokers typically experience an increased urge to smoke when taking part in weight loss program, mainly since nicotine lowers appetite and decreases the taste for food. Unexpectedly some smokers having taken thylakoid shoots regularly spontaneously have reported lower need for nicotine. Typically, thylakoid shoots are provided as part of a weight loss program.

By mistake a smoker was included in the study of example 1. This woman, who had smoked about 17 cigarettes a day for the last 40 years, reported significantly lower urge to smoke once taking part in the study. Once the study was over she reported that the urge to smoke had increased again.

Another woman, typically smoking about 23 cigarettes per day, reported that her consumption of cigarettes was reduced to about 15 cigarettes per day once she started taking thylakoid shoots regularly. Further, she reported a lower urge to smoke, which she believed to be the explanation to why she all the suddenly was smoking less.

A man, typically smoking 15 to 20 cigarettes a day, reported that his consumption of cigarettes was reduced to less than 10 cigarettes per day - typically to about 9 cigarettes per day - 10 days after he had started taking thylakoid shoots regularly in the morning.

### Example 3 - decreased urge for palatable food during dietary intervention

The effects observed in example 1 were achieved without any caloric restriction. However, most weight loss programs are based on a caloric restriction. Hence, the effect of thylakoids on hedonic hunger during a caloric restriction commonly used in weight loss studies was also studied.

### Subjects

Forty-eight middle-aged (40-65 years) non-smoking overweight women who were not, currently or recently, on a diet were recruited for screening through advertisement in the local newspaper. Exclusion criteria were diabetes, inflammatory bowel disease, thyroid disease, food allergies and a BMI over 33. Thirty individuals were enrolled as participants in the study. All of the 30 enrolled participants finished the study. Compliance was measured through diary entries, interviews and the daily use of pedometers. Four participants (one from the control group and three from the thylakoid group) were excluded from the data analysis due to incompliance with the diet and exercise regime.

Baseline characteristics of the 26 included subjects are listed in **Table 1.** One participant in the control group was not included in the analysis of subjective ratings of hunger, satiety and urges for specific food items due to non-attendance.

**Table 1. Baseline characteristics of the 26 women included in the study (average ± SD). There were no significant differences between the groups.**

| | **Control group (n= 14)** | **Thylakoid group (n= 12)** |
|---|---|---|
| **Age (years)** | 53.0 ± 5.9 | 51.3 ± 5.3 |
| **Weight (kg)** | 76.6 ± 5.3 | 73.1 ± 7.4 |
| **BMI (kg/m²)** | 27.7 ± 2.2 | 27.4 ± 1.7 |
| **Waist circumference (cm)** | 88.9 ± 6.6 | 85.7 ± 8.3 |
| **Hip Circumference (cm)** | 103.4 ± 4.3 | 103.3 ± 4.5 |
| **Waist/Hip ratio** | 0.86 ± 0.06 | 0.83 ± 0.06 |

Procedures, objectives and requirements of the study were explained in detail to the participants, and written consents were signed both before screening and before the study started. The study was approved by the Ethical Committee of Lund University, and conducted in accordance with the declaration of Helsinki. After completing the study, all participants received a compensation of 2000 SEK (taxable).

### Experimental study design

The study was conducted and designed as a single-blinded, randomized, diet and exercise intervention study with duration of two months. The participants were randomized into two groups to ensure normal distribution within and between the groups based on body weight, BMI, blood glucose, insulin, triacylglycerides (TAG) and cholesterol (total and LDL). One of the groups received supplementation by thylakoids in a blueberry drink every day, while the other group served as control, receiving a daily blueberry drink without thylakoids.

Every second week, at the same time in the morning, the participants visited the clinic for measurements of body weight, body composition, waist and hip circumferences and for blood sampling. To optimize conditions for all measurements, the participants were instructed to have a standardized dinner in the evening before each test day and to abstain from further intake of foods or liquid after 8.00 pm.

The participants had a total of five individually scheduled appointments during the study. On the first and the last day (day 1 and 56), following the anthropometric measurements and blood sampling, an isocaloric breakfast (2114 kJ/505 kcal) consisting of the blueberry drink with or without thylakoids, yoghurt with apple, breakfast cereal, nuts and coffee or tea was served (**Table 2**). After 240 minutes an isocaloric take-away lunch (2593 kJ/630 kcal) consisting of a frozen thai-curry meal, bean salad and a banana was administered. VAS questionnaires measuring subjective parameters of hunger, satiety and urge for specific foods were filled out at given time points throughout the day on both day 1 and 56, whereas blood samples were taken only during the initial 240 minutes following breakfast.

**Table 2. Contents of breakfast and lunch served on first and last days of the study.**

| **Meal components** | **Amount (g)** | **Calories (kJ/kcal)** | **Protein (g)** | **Fat (g)** | **Carbohydrates (g)** | **Fiber (g)** |
|---|---|---|---|---|---|---|
| **Breakfast** | | | | | | |
| Natural yoghurt | 300 | 753 / 180 | 10.2 | 9.0 | 14.4 | 0.0 |
| Muesli wt. fruits and nuts | 45 | 775 / 185 | 2.9 | 4.9 | 30.4 | 3.4 |
| Apple (Granny Smith) | 60 | 139/34 | 0.2 | 0.1 | 7,4 | 1.1 |
| Almonds | 10 | 255 / 61 | 2.0 | 5.2 | 1.3 | 0.7 |
| Blueberry shot, wt or wth thylakoids | 50 | 192/45 | 2.7 | 3.4 | 5.5 | 0.5 |
| *Total intake* | *465* | *2114* / *505* | *18.0* | *22.6* | *59.1* | *5.7* |

| **Lunch** | | | | | | |
|---|---|---|---|---|---|---|
| Red Curry Chicken (frozen product) | 380 | 1558 / 380 | 15.2 | 13.3 | 45.6 | 1.9 |
| Bean salad (ready product) | 100 | 591 / 144 | 6.0 | 5.0 | 18.0 | * Not stated on product |
| Banana | 105 | 444 / 106 | 1.0 | 0.5 | 23.1 | 1.8 |

### Thylakoids

The thylakoids used in the present study were prepared from baby spinach leaves using the pH-method, as described (Emek SC, et al A large scale method for preparation of plant thylakoids for use in body weight regulation, Prep Biochem Biotechnol 2010, 40(1):13-27). The thylakoid-slurry was dried to obtain a thylakoid powder, prepared by Swepharm AB (Södra Sandby, Sweden). 100g thylakoids consists of 41.1 g protein, 14.5 g fat, 36.8 g carbohydrate, 3.5 g salt as well as pigments such as 3640 mg chlorophyll, 28 mg lutein, 730 ug zeaxantin, 4 760 ug betakaroten, 21 ug vitamin A, 1330 ug vitamin K, 6.07 mg vitamin E and 166 ug folic acid.

The thylakoid group received 5.6 g of thylakoid powder mixed with 2.8 g rapeseed oil (Zeta, Di Luca & Di Luca AB, Stockholm, Sweden) and 50 g of blueberry soup (Ekströms original, Procordia Food AB, Eslöv, Sweden). The control group received 2.8 g rapeseed oil mixed with 50 g blueberry soup. The blueberry drinks with and without thylakoids contained 209 kJ/50 kcal versus 188 kJ/45 kcal respectively. The drinks were taken before breakfast every day.

### Caloric restriction and diet recommendations

Before the study started, average daily energy requirement for the participants was calculated to 8800 kJ (∼2100 kcal), with respect to age, weight, height and presumed daily energy-consumption, according to Harris Benedict equation using Dietist XP (Kostdata, Bromma, Sweden). During the study the calculated energy intake was reduced by 15 E% to ∼7500 kJ/day (∼1800 kcal/day). The participants were provided with a collection of selected recipes (3 breakfasts, 29 lunches/dinners and 4 desserts) to choose from. The recommended energy intake per day during the study was divided into three meals/day: 2100 kJ (∼500 kcal) for breakfast, and 2500 kJ (∼600 kcal) for lunch and dinner respectively. An additional 400 kJ (∼100 kcal) was allowed for milk in coffee/tea and individual adjustments. Water, coffee and tea were allowed between meals, but no additional foods or snacks. The diet did not allow any sweetened drinks.

Subjects were also instructed to accomplish 60 minutes of low/medium intensity exercise each day, such as power walking, swimming, basic aerobics etc.

Each day, the participants answered questions regarding their choice of meals, health-status and exercise in a diary. These data were used to analyze the compliance with the study guidelines.

### Somatic analyses

Body weight was measured with a digital scale (TANITA WB-100A, class III, Amsterdam, The Netherlands). The composition of body fat mass (kg) and trunk fat (kg) were measured with a Bioelectric Impedance Analyser (TANITA-BC 418 MA). Waist circumference, midway between the lower rib margin and the iliac crest, and hip circumference were measured to the nearest 0.5 cm by using a non-stretchable tape measure.

### Questionnaires

Questionnaires constructed as VAS (Flint A, et al Reproducibility, power and validity of visual analogue scales in assessment of appetite sensations in single test meal studies, Int J Obes Relat Metab Disord 2000, 24(1):38-48.) were used to measure sensations of hunger, fullness and urge for specific food items. Pictures assisted the evaluation of the urge for specific food items. For high carbohydrate snack pictures of a sandwich and a sweet cinnamon bun were presented, for salt and fat; pictures of potato chips and salted peanuts, for sweet snack; pictures of candy and a popsicle and for fat and sweet; pictures of cake and chocolate were presented. First (day 1) and last day (day 56) of the study, subjects answered questions before breakfast (0 min) and at time points 15, 60, 120, 180, 240 (before lunch was served), 270 (after lunch was served), 330, 390, 450 and 630 minutes. Written instructions were given on the front page of the questionnaire, and each subject was individually instructed in how to fill out the questionnaire to avoid misinterpretation. Questions were followed by a 100 mm line anchored by descriptors on each side of the line, see **Table 3.** Subjects were instructed to place a vertical line across the scale, thus rating how strong their sensations were at every time point. Ratings were scored as mm between "not at all" and the individual subjects mark.

**Table 3. Questions in the VAS-questionnaires asked during the first and last days of the study.**

| **Questions** | **Anchor: 0 mm** | **Anchor: 100 mm** |
|---|---|---|
| **How hungry are you right now?** | Not hungry at all | Extremely hungry |
| **How full are you right now?** | Extremely full | Not at all full |
| **How much would you like to have a sandwich or a cinnamon bun right now?** | Not at all | Extremely much |
| **How much would you like to have salted peanuts or chips right now?** | Not at all | Extremely much |
| **How much would you like to have sweets/candy right now?** | Not at all | Extremely much |
| **How much would you like to have chocolate right now?** | Not at all | Extremely much |

### Statistics

Power calculations were based on previous pilot-studies examining thylakoid supplementation in humans with respect to changes in blood-glucose. With a sensitivity of 0.80 and a significance level of 0.05, the power calculations indicated a sample size of 14 in each group, when the clinical difference was set to 0.6 and the within-subject standard deviation of 0.56. Statistical data analyses of all blood samples and body measurements were done using R Development Core Team, version 2.15.3, 2011 (R Foundation for Statistical Computing, Vienna, Austria). The analysis was performed in two steps. First, the simple regression model was fitted for each subject and for each of the 13 measured variables by taking time as explanatory variable (5 time points) and the measured variable as the response variable. The obtained slope values represented fitted rate of change for a particular individual and variable. Second, multivariate analysis was performed on the so obtained rates of change, using two-sample Hotelling's T2-test with all 13 variables treated together. Deviations from the normality assumption were examined and were determined not to be severe. Additionally, for interpretation purposes, the mean difference in slope variables between the two groups were analyzed with the t-test for a univariate two-sample problem for each of the 13 measured variables. The obtained p-values from these individual variable comparisons should be treated with caution due to the effect of multiple testing and thus are only used to discuss and interpret which of the variables contribute most to the significant difference between the groups.

Statistical analyses of the VAS questionnaires were done using Prism, version 6 (GraphPad Software, Inc, San Diego, CA, USA). On the first and last days respectively, the variations in ratings during the day were analysed with a two-way repeated measures ANOVA with treatment and time as fixed factors. Individual time points were further analysed with a multiple comparison test followed by Fisher's LSD test. Numerical calculations of total area under the curve (tAUC) were analysed with Wilcoxon matched-pairs signed ranks test to compare the difference between first and last days of the study within the groups. The Mann-Whitney t-test was also used to compare differences between the groups.

Objective data exhibited normal distribution for most variables and some deviations from normal assumption for certain variables (HbA1c, Apo B and body weight in the thylakoid group), but not critical for the result of the analysis. The latter was assessed by a re-sampling study. In the figures, data are expressed as mean ± SEM, while in the tables data are given as mean ± SD. P-values <0.05 were considered to be statistically significant, and p-values <0.1 to be of interest.

### Results

After two months of restricted diet all participants lost body weight and decreased their total body fat with no significant difference between the control and thylakoid treated groups.

Analysis of subjective ratings of hunger, using VAS questionnaires, revealed a decreased sensation of hunger within the thylakoid group at the end of the study compared to the first day (p=0.016, Fig. 3A), whereas no change of hunger sensation was found in the control group (Fig 3A). No differences in hunger sensations were found between the thylakoid group and control on the first day (F(10,240)=1.6, ns) or on the last day of the study (F(10,210)=0.83, ns) (Figs. 3B and 3C).

A strong tendency for a reduction in the urge for chocolate within the thylakoid group was observed at the end of the study compared to the first day (p=0.052), but not in the control group (p=0.62, Fig 4A), confirming that the thylakoids, not the dietary intervention, reduces the urge for chocolate. The ANOVA analysis of the urge for chocolate between treated and control on the first day and the last day respectively revealed a significant interaction between time and treatment (first day; (F(10,240)=1.9, p<0.05), last day (F(10,230)=1.9, p<0.05). Analysis of individual time points showed a decreased urge for chocolate in the treatment group prior to lunch on the first day and in the afternoon on the last day (Figs. 4B and 4C).

The urge for a carbohydrate snack was not significantly altered over the course of the study in any of the groups (Fig. 5A). There was however an interaction between time and treatment in the urge for a carbohydrate snack on the first day of treatment (F(10,240)=2.1, p<0.05), but not on the last day (F10,230)=1.3, ns, fig 5C). The urge for a carbohydrate snack was however decreased prior to lunch and in the afternoon on the first day (Fig. 5B). The results show that both thylakoids and dietary intervention reduces the urge for carbohydrate snacks. Further, the findings of Fig. 5B confirms that findings of example 1.

No side effects of the thylakoid supplementation were reported.

### Discussion

Daily supplementation of thylakoids for two months in combination with a restricted diet resulted in a body weight loss of similar magnitude in the thylakoid-treated and the control groups. The overall decrease in body weight was 0.65 kg/week in the thylakoid group and 0.59 kg/week in the control group (difference not significant).

Feelings of hunger and urge for chocolate were reduced by thylakoid treatment over the course of the study. In contrast, they were not reduced in the control group. Furthermore, the urge for chocolate and a carbohydrate snack was decreased in the thylakoid treated group compared to controls on the first day of the study and for chocolate also on the last day.

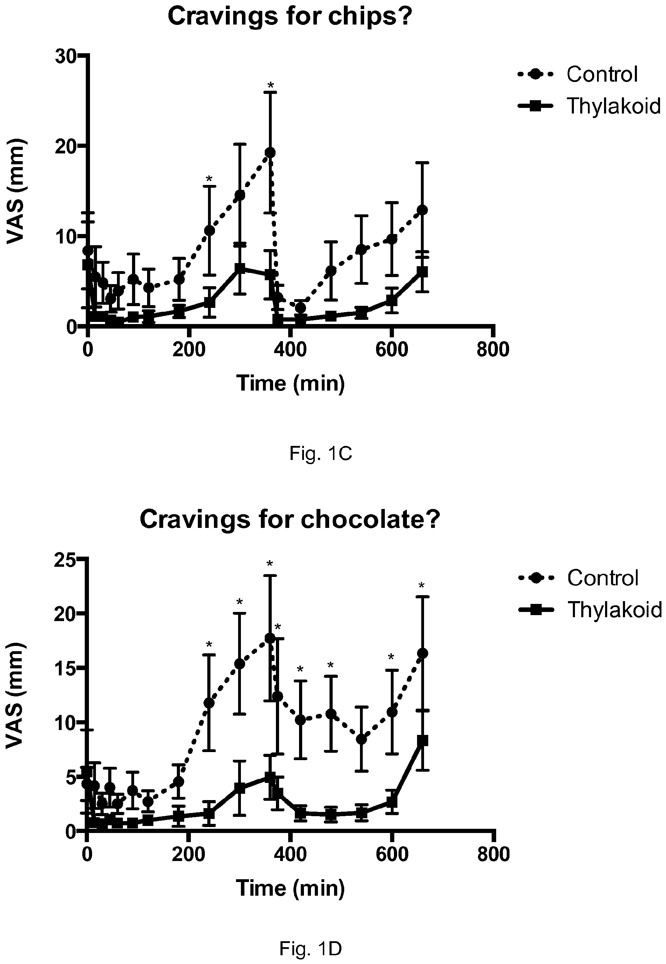

Interestingly, on the first day (cf. Fig 3B) no difference in feelings of hunger (homeostatic hunger) was seen, while both the urge for chocolate (cf. Fig. 4B) and carbohydrate snack (cf. Fig. 4C) was reduced. This is in line with the findings of example 1 (cf. Fig. 1). Clearly, thylakoids has an effect on hedonic hunger, being distinct from its long term effect on homeostatic hunger, confirming the effect of the present invention. Further, as is apparent from example 1 and 3, thylakoids are able to suppress hedonic hunger, irrespective of caloric restriction.

Even though the body weight reduction was similar between the two groups, it would appear that this level of weight loss was reached with less effort in the thylakoid-treated group compared to the control group, based on the reduced subjective ratings of hunger and urge for palatable food in the thylakoid group. This suggests that thylakoids exert their appetite controlling effect even during caloric restriction and that this effect is sustained following weight loss. This is an important property of thylakoid treatment, since hunger is common upon weight loss and often leads to overeating and body weight regain.

Thus, intake of thylakoids alleviates some of the strains coupled to caloric restriction during body weight loss programs. Further, intake of thylakoids assists in adopting a more healthy eating behavior and reducing the relative intake of palatable food. This may be crucial in maintaining a healthy eating behavior and avoiding weight gain subsequent to a weight loss program.

Thus it may be concluded that isolated thylakoids are able to suppress hunger hedonic hunger irrespective of caloric restriction.

### Example 4 - suppression of wanting and hedonic hunger in emotional eaters

### Materials and methods

### Subjects

32 women were recruited through local advertising. The volunteers were assessed for eligibility through a screening procedure including questionnaires evaluating general health, eating behaviours and subjective liking for different food products. During the screening process, 6 women were excluded and 26 women included in the study. Inclusion criteria: women ages 40-70, normal weight or overweight. Exclusion criteria: Diabetes, illnesses affecting appetite, food allergies or intolerance to food served in the study, or dieting during the last 3 months. 22 subjects completed the study and were included in the final analysis (Table 1).

**Table 1 - Baseline characteristics**

| | | Median | Interquartil range | Range (min-max) |
|---|---|---|---|---|
| Age (years) | (n=22) | 54.5 | 47.0-59.5 | 40-66 |
| BMI (kg/m²) | (n=22) | 25.3 | 24.5-29.4 | 22.4-36.2 |
| Body fat (%)^{a} | (n=21) | 37.2 | 31.5-40.4 | 26.9-47.1 |

| | | | | |
|---|---|---|---|---|
| ^{a} Body composition analyzer (TANITA-BC 418 MA, Amsterdam, The Netherlands. One subject excluded from this analysis due to failure of the body composition analyser. | | | | |

### Study design

The study was a randomised, placebo-controlled, double blind, single-centred meal intervention study with a cross over design, conducted at the Biomedical Centre (BMC) at Lund University, Sweden. Each participant was tested on two days, separated by a wash out period of at least 1 week, receiving a green-plant supplement (5 g) on one day and placebo on the other. The allocation to treatment or placebo was randomised and balanced between test days.

Subjects arrived in the morning, fasting from 22:00. During test days a schedule was followed as shown in table 2. No alcohol or intense physical activity was allowed the days before or during test days. Before breakfast, the participants filled out a questionnaire constructed as Visual Analogue Scales (VAS) (Flint et al. Int J Obes Relat Metab Disord, 25, 781-792) with questions regarding hunger, satiety and wanting for specific food products. Subsequently, a blueberry drink, with or without supplementation of green-plant membranes (thylakoids), was served followed by a standardized high carbohydrate Swedish breakfast. The VAS-questionnaire was filled out every hour as well as before and after lunch and the *ad libitum* snack buffet, served at 13:00 and 16:00 respectively. Between breakfast and lunch, participants left the premises for work or free activities, and were reminded to fill out the VAS-questionnaires every hour via text messages. After lunch, the participants stayed in their allocated seats, sitting side by side with cardboard dividers between them, all facing a window. They were allowed to read or work on laptops until 16:00 when the snack buffet was served at the table on individual trays. A leaflet, containing information about the products served, accompanied the buffet, ensuring the freshness of the foods. It included names of manufacturers and pictures of the packaging. The same pictures were used for the VAS-questionnaire. Before starting to eat from the snack buffet in front of them, the participants filled out the VAS-questionnaire once more. Then they were allowed to eat freely from the products on their trays, encouraged to follow their urges and ask for more should they run out. They were occupied by the snack buffet for 45 min, not doing anything else during this time. Afterwards, they filled out the VAS-questionnaire again, this time including questions regarding how much they liked the products they tasted. Finally, they had the opportunity to comment freely on how they felt during the test day.

**Table 2 - Test day schedule, day 1 and 2**

| Time | Event | Timepoint |
|---|---|---|
| 07:30 | Participants arrived at BMC | |
| 07:45 | VAS | Baseline; Time point 0 |
| 08:00 | Blueberry drink, with/ without 5 g green-plant membranes (treatment/placebo) Breakfast, including coffee/tea. | |
| 08:15 | VAS | Time point 15 min |
| 09:00 | VAS | Time point 60 min |
| 10:00 | VAS | Time point 120 min |
| 11:00 | VAS | Time point 180 min |
| 12:00 | VAS | Time point 240 min |
| 12:45 | VAS | Time point 285 min |
| 13:00 | Lunch | Time point 300 min |
| 13:20 | VAS | Time point 320 min |
| 14:00 | VAS | Time point 360 min |
| 15:00 | VAS | Time point 420 min |
| 16:00 | Snack buffet served. VAS | Time point 480 min |
| 16:45 | VAS after snack buffet | Time point 525 min |

### Power and sample size

The number of participants required to achieve a power of 0.8 in this crossover study was calculated using data from an earlier study. Based on these calculations, 15 subjects were needed to detect a 10 mm difference in VAS-ratings of hunger. This number of subjects is also sufficient for detecting differences in ratings for wanting palatable food. According to literature, 17 subjects should be sufficient for detecting a difference in food intake of 500kJ/120kcal.

### Ethics

The study was approved by the Ethics Committee for Human Studies in Lund (2006/361). The trial was conducted in accordance with the Declaration of Helsinki. All subjects gave written informed consent before the study began. The participants did not receive any monetary compensation.

### Green-plant membranes

The green-plant membranes (Appethyl, Greenleaf Medical AB, Stockholm, Sweden) used in the present study were prepared from baby spinach leaves using the pH-method as previously described (Emek et al., Prep Biochem Biotechnol, 40, 13-27), followed by drum drying. 100 g of green-plant membranes contain 26.1 g protein, 7.24 g fat, 48.7 g carbohydrate, 27.9 mg lutein, 730 µg zeaxantin, 3.45 mg betakaroten, 21 µg vitamin A, 1330 µg vitamin K₁, 6.07 mg vitamin E and 166 µg folic acid. 5 g green-plant membranes were served in a cold blueberry drink, mixed with 2.5 g rapeseed oil (Zeta, Di Luca & Di Luca AB, Stockholm, Sweden) and 92.5 g blueberry soup (Ekströms original, Procordia Food AB, Eslöv, Sweden), served immediately before breakfast on treatment test days. On control test days, the participants were served a placebo drink, which consisted of 2.5 g rapeseed oil mixed with 92.5 g blueberry soup. The blueberry drinks contained 82 kcal with green-plant membranes and 64 kcal without.

### Meals

Breakfast on both test days was a standardized Swedish high carbohydrate breakfast with yoghurt and muesli, one slice of white bread with butter, cheese and sweet pepper, coffee or tea. The energy distribution was 31.2 per cent of total energy (E%) fat, 15.4 E% protein and 53.4 E% carbohydrates. Sugars constituted 22.6 E%. The lunch served on both test days was a slice of pizza, made from sourdough, with tomato sauce, ham, onion, mushrooms and cheese, served with boiled broccoli and tap water. Coffee or tea following lunch was optional, but subjects were required to have the same both test days. Energy distribution of the lunch was 45.9 E% carbohydrates, 32.5 E% fat and 21.6 E% protein. In addition, participants were allowed one cup of coffee/tea, without sugar or sweetener, between breakfast and lunch, and one cup of coffee/tea after lunch. Calculations of energy distribution were made using Dietist Net Pro (Kost och Näringsdata AB, Bromma, Sweden).

### Snack buffet

A range of bestselling snacks, sweets and confectionery was chosen for the snack buffet (Table 3), based on the responses to the product questionnaire. These products were grouped in three different categories: salty, sweet, and sweet-and-fat snacks. Snacks with salty taste included potato chips and salted assorted nuts, products with sweet taste, containing sugar, included two different kinds of sweets and orange juice, products with sweet taste containing both sugar and fat were: milk chocolate, dark chocolate, chocolate covered toffee, chocolate pastry, and cinnamon bun. Each participant received a tray with the products served in separate containers, weighed before and after consumption. The amounts were chosen to be more than enough for one person, to ensure ad libitum consumption. Coffee, tea and water were served with the snacks in a limited amount. The drinks were optional, but the subjects had to have the same on both test days.

**Table 3 - Components of the snack buffet, energy content per 100g**

| Products | Calories (kJ/kcal) | Protein (g/E%) | Fat (g/E%) | Carbohyd r. (g/E%) | Sugar (g/E%) | Salt (g) |
|---|---|---|---|---|---|---|
| **Salty** | | | | | | |
| **Potato chips** "Sourcream &Onion" (Estrella, Sweden) | 2200/525 | 6.0/4.6 | 32.5/56.4 | 50.5/39 | 3.6/2.8 | 1.7 |
| **Assorted nuts** "Vår klassiska nötmix" (OLW, Sweden) | 2450/590 | 25.0/16.8 | 48.0/72.5 | 16.0/10.7 | 4.2/2.8 | 1.3 |

| **Sweet** | | | | | | |
|---|---|---|---|---|---|---|
| **Sweets** "Ahlgrens bilar" (Cloetta, Sweden) | 1450/350 | 6.0/6.8 | 0.3/0.8 | 81.0/92.4 | 55.0/62.7 | 0.25 ^{a} |
| **Sweets** "Gott&Blandat" (Malaco, Sweden) | 1450/340 | 0.0/0 | 0.0/0 | 85.0/100 | 61.0/71.8 | 0.25 ^{a} |
| **Orange juice** "Apelsin juice" (Kiviks Musteri AB, Sweden) | 180/40 | 0.6/5.9 | <0.5/11 | 8.5/83.1 | 8.5/83.1 | 0.01 ^{a} |

| **Sweet-and- fat** | | | | | | |
|---|---|---|---|---|---|---|
| **Milk chocolate** "Marabou mjölkchoklad", (Marabou, Sweden) | 2290/550 | 4.8/3.5 | 32.0/53 | 59.0/43.4 | 58.0/42.7 | 0.25 ^{a} |
| **Dark chocolate** "Lindt Excellence 70% dark" (Lindt & Sprüngli, Sweden) | 2180/520 | 8.0/6.1 | 40.0/68.7 | 33.0/25.2 | 28.0/21.4 | 0.15 ^{a} |
| **Chocolate covered toffee** "Dumlekola original" (Fazer, Sweden) | 1980/470 | 3.6/3.1 | 21.0/40.4 | 66.0/56.5 | 53.0/45.4 | 0.4 ^{a} |
| **Chocolate pastry** "Delicatoboll", (Delicato, Sweden) | 2000/480 | 5.0/4.2 | 29.0/54.7 | 49.0/41.1 | 30.0/25.2 | 0.4 |
| **Cinnamon bun** "Kanelbulle" (Bonjour, VAASAN Sverige AB, Sweden) | 1350/320 | 6.2/8 | 9.6/27.8 | 50/64.3 | 15/19.3 | 0.5 |

| | | | | | | |
|---|---|---|---|---|---|---|
| ^{a} Salt content initially expressed as sodium content has been converted to amount of salt by multiplying the sodium value by 2.5, as recommended by the Swedish national food agency (Livsmedelsverket). | | | | | | |

### Product questionnaire

The product questionnaire was constructed for this study as a tool for choosing well-suited products to measure cravings and snack intake in a Swedish population. It contained specific questions about snacks of the most popular brands in Sweden: potato chips, nuts, chocolate, sweets, baked confectionery, buns, fruit juice and soft drinks. Questions were asked about how often the responder consumed the palatable foods, how much they liked the products on a nine point hedonic scale and which product in each category they preferred.

### Three-factor eating questionnaire revised 18-item, version 2 (TFEQ-R18V2)

The TFEQ-R18V2 is a revised version of the original Three-Factor Eating Questionnaire (TFEQ), a self-assessment scale widely used in studies of eating behaviour. While the original TFEQ contained 51 items, TFEQ-R18V2 contains 18 questions to measure three types of eating behaviour: emotional eating, uncontrolled eating and cognitive restraint. The questionnaire used in the present study was a Swedish translation.

### VAS-questionnaire

Questionnaires constructed as visual analogue scales VAS were used repeatedly during test days (Table 2), to measure sensations of appetite and cravings for specific food items (Table 3). The questionnaire was constructed as a booklet with written instructions on the first page. All questions were followed by a 100-mm horizontal line, on which subjects marked their response. The line was anchored at each end, expressing the minimum value on the left: "Not at all", and the maximum value on the right: "Extremely". At the last time point of the day, questions were added about liking for the products tasted that particular day, to enable evaluation of the rewarding properties of the items. When analysed, ratings for the individual food items were merged into three different categories and presented as salty, sweet, and sweet-and-fat snacks (Table 3). For analyses regarding all snacks together, all values were merged and presented as total/all snacks.

### Statistics

All statistical analyses were done using the Prism version 6, statistical software (GraphPad Software, Inc, San Diego, CA, USA). Normal distribution of the paired parameters and the calculated differences between treatment and control conditions was computed by d'Agostino & Pearson omnibus normality test, verified by boxplot and histogram analysis and comparison between mean and median values. Due to the small population sample, normal variation was hard to determine, which is why non-parametric tests were preferred. Variations in VAS-ratings over time were analysed with a two-way repeated measures (RM) ANOVA in order to test time, treatment, and time by treatment interaction. Analyses of VAS-ratings at individual time points as well as differences in total area under the curve (tAUC) for VAS-ratings, food intake and ratings of liking were performed using Wilcoxon matched-pairs signed rank test. Given *p*-values in text and figure legends were analysed with Wilcoxon matched-pairs signed rank test if not otherwise stated.

Treatment effect of green-plant membranes on feelings of hunger, satiety and urge for specific food products and liking respectively was calculated by subtracting VAS-ratings for treatment day from control day values, except for satiety, which was calculated the opposite way. Treatment effect on food intake was calculated as difference in caloric intake between control and treatment days. Calculating delta values for the treatment effects was required to account for paired observations. These delta values were then correlated to eating behaviour scores, as measured by the TFEQR18-V2 questionnaire, divided into the three domains: emotional eating, uncontrolled eating and cognitive restraint. Correlations were also calculated between eating behaviour scores, BMI and body fat percentage. All correlations were computed by Pearson correlation coefficient. Correlation coefficients (r), and coefficients of determination, (R²), equal to and above 0.3 were considered fairly strong and have been included in the manuscript. In both figures and text, data are expressed as mean +/- SEM if not otherwise stated. *p*-values <0.05 were considered statistically significant, and *p*-values <0.10 of interest.

### Results

### VAS-ratings on hunger and satiety

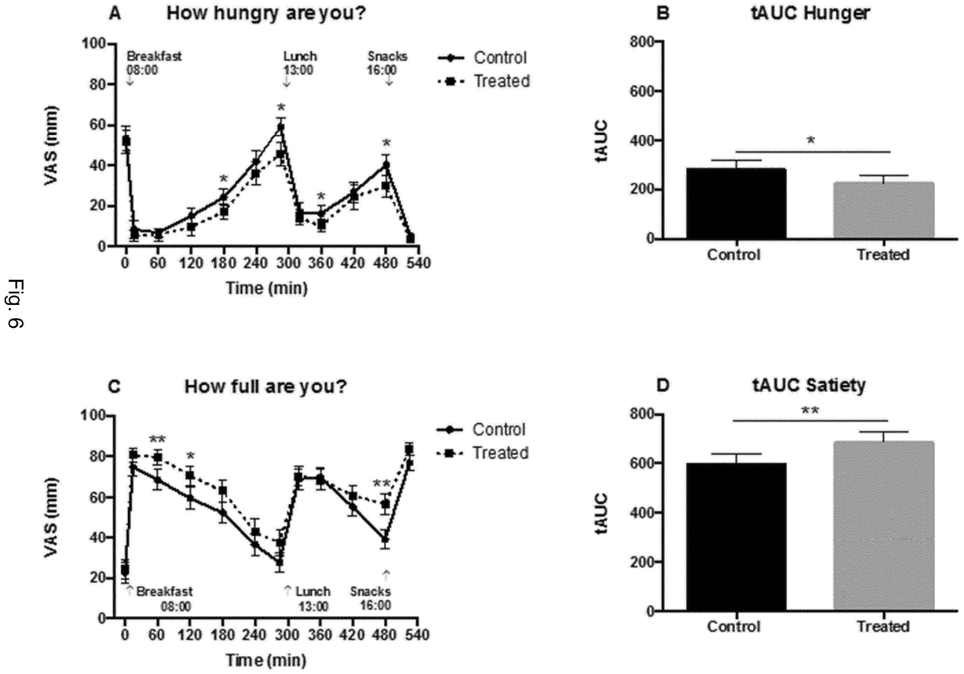

Treatment with green-plant membranes reduced subjective ratings of hunger compared to control and were lower throughout the day (F (1,21) = 6.237, *p*<0.05, Fig. 6A), the tAUC for hunger decreased by 21% between control and treated conditions (p<0.05, Fig. 6B). The difference in hunger ratings between control and treatment was most pronounced prior to lunch and before the snack buffet, reaching highest significance at time points 285 min and 480 min after beginning of breakfast (Fig. 6A).

Treatment with green-plant membranes also increased ratings of satiety compared to control (Fig. 6C-D). Satiety-scores were generally higher during the whole day in the treated condition (F (1,21) = 8.745, *p*<0.01, Fig. 6C), tAUC for satiety being increased by 14% following treatment (*p*<0.01, tAUC, Fig. 6D). Significant differences in satiety between control and treatment groups were observed at 60 and 120 min, as well as immediately before the snack buffet at time point 480 min (Fig. 6C).

### VAS-ratings on wanting palatable foods

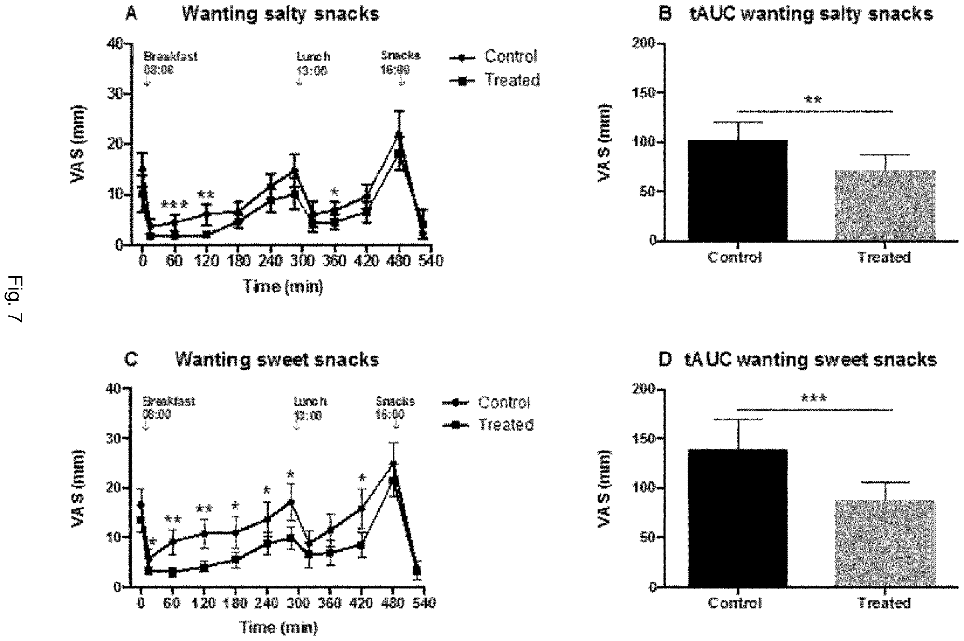

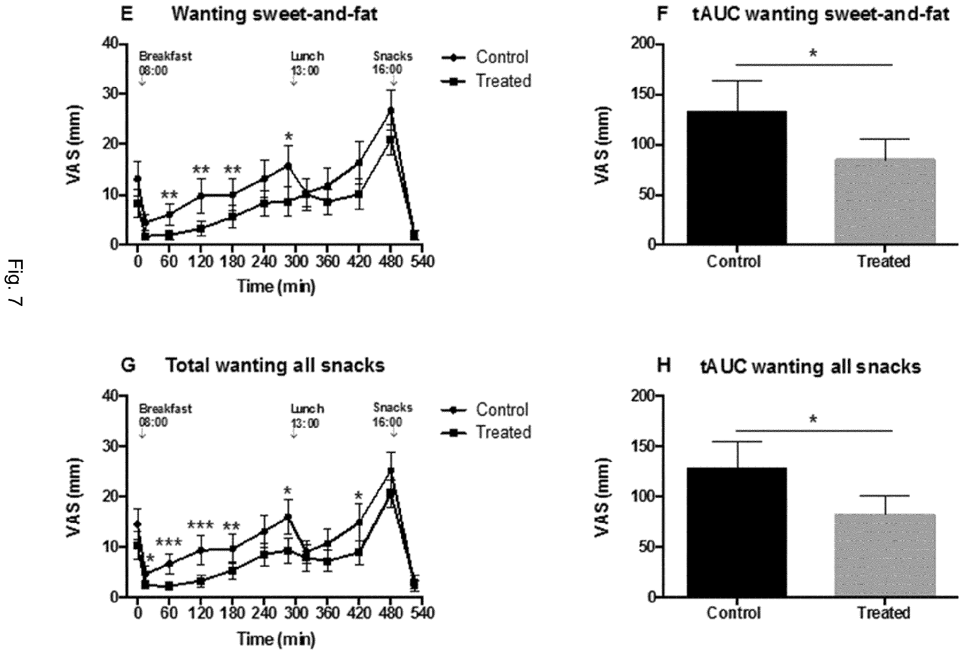

Treatment with green-plant membranes decreased subjective feelings of wanting all kinds of palatable food compared to control (Fig. 7). VAS-ratings were consistently lower during the treatment test day for all categories of snacks (salty, sweet and sweet-and-fat) as well as total wanting for all snacks.

Effect of treatment on ratings for wanting salty snacks is presented in Fig. 7A (F (1,21) = 6.110, *p*< 0.05). Treatment reduced wanting for salty snacks by 30% between control and treated conditions (*p*<0.01, tAUC, Fig. 7B). Specifically, the urge for salty snacks was lower in the treated group at time points 60, 120 and 360 minutes compared to control (Fig. 7A).

Effect of treatment on the ratings for wanting sweet snacks is presented in Fig. 7C (F (1,21) = 8.412, *p*<0.01). Wanting for sweet decreased 38% between control and treated conditions (*p*<0.001, tAUC, Fig. 7D). Furthermore, there was a tendency towards a time by treatment interaction in wanting sweet snacks, i.e. the time impact on VAS-ratings depended on whether the subject received treatment or placebo (F (11,231) = 1.803, *p*=0.05). The urge for sweet snacks was lower in the treated group compared to control at all time points between breakfast and lunch, starting already at 15 minutes after breakfast. It was also lower in the afternoon at time point 420 minutes (Fig. 7C).

Effect of treatment on the ratings for wanting sweet-and-fat snacks is presented in Fig. 7E (F (1,21) = 5.198, *p*<0.05). Wanting for sweet-and-fat decreased 36% in the treated group compared to control (*p*<0.05, tAUC, Fig. 7F). The urge for sweet-and-fat snacks was lower in the treated group compared to control at time points 60, 120, 180 minutes and also right before lunch at 285 minutes (Fig. 7E).

The ANOVA analysis of total wanting of all snacks revealed an effect of treatment (F (1,21) = 7.364, *p*<0.05, Fig. 7G). Subsequent analysis of individual time points showed that treatment decreased total wanting at 15, 60, 120, 180, 285 and 420 minutes after breakfast (*p*<0.05). Wanting for all snacks combined decreased by 36% in the treated group compared to control (*p*<0.05, tAUC, Fig. 7H).

### Food intake from ad libitum snack buffet

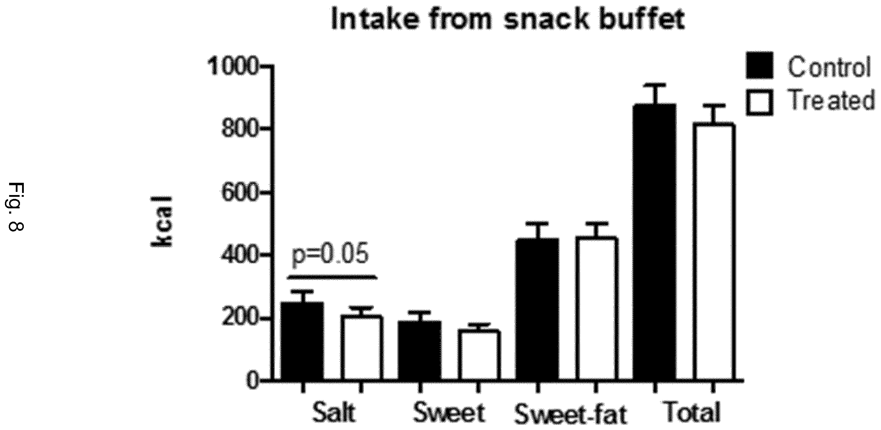

Of the 22 subjects, everyone ate something from the snack buffet on both test days. There was a strong tendency towards a reduced caloric intake of salty food items following treatment with green-plant membranes compared to control, with a median difference of 65 kcal (*p*=0.0547, Fig. 8). There were no significant differences in the intake of sweet or sweet-and-fat snacks, or in the total consumption between treatment and control conditions.

### Liking for palatable food, measured after consumption from the snack buffet

Only the subjects who tasted an item were allowed to rate their liking for it. For each specific food product to be included in the analysis of liking scores, subjects had to taste and rate their liking for the product at both control and treatment days. One individual was excluded from the analysis due to failure to fill out the form correctly. Treatment with green-plant membranes produced a lower liking of sweet products after consumption, compared to control days (*p*<0.01, Fig. 9). In contrast, the liking for salty and for sweet-and-fat products was unchanged between treatment and control days. There was no significant difference in total liking for all snacks on treatment days compared to control days (*p*=0.15, Fig. 9).

### Eating behaviour measured by the Three-Factor Eating Questionnaire (TFEQ-R18V2)

Participants responded to each of the 18 questions about statements characterizing eating-related behaviours on a four-point Likert scale. Responses were coded on a four-point scale (1-4) with higher values indicating more of the behaviour. Mean values for each of the three behavioural categories were: emotional eating domain 2.27 (+/- 0.15), cognitive restraint domain 2.52 (+/- 0.15) and uncontrolled eating domain 2.26 (+/- 0.10).

### Correlations between treatment effect on wanting and scores for eating behaviour

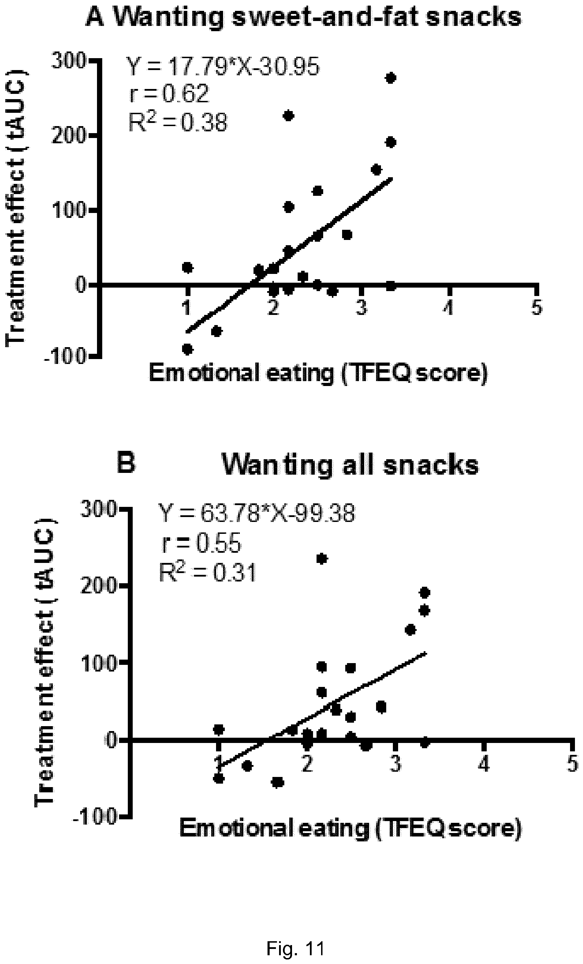

The treatment effect on wanting sweet-and-fat snacks as well as all kinds of snacks showed positive correlation with emotional eating scores, so that higher scores for emotional eating behaviour was correlated to a greater treatment effect of green-plant membranes on ratings for wanting palatable food in general, and sweet-and-fat foods in particular (p<0.01 respectively, Pearson r, Fig. 10A and B). Cognitive restraint and uncontrolled eating domains failed to show any correlation between treatment effects and score for eating behaviour.

### Additional correlations analysed

There were no correlations between the different eating behaviour scores and the treatment effect of green-plant membranes on hunger, satiety, food intake or liking. Neither were there any correlations between BMI or body fat percentage and scores for eating behaviour, nor any correlations between the different eating behaviour scores.

No adverse events or effects were reported.

### Discussion

The present example demonstrates that intake of thylakoids prior to breakfast increase subjective ratings of satiety and decrease ratings of hunger as well as cravings for snacks and sweets during the day. When correlated to eating behaviour scores, treatment effect of green-plant membranes on wanting was positively correlated to emotional eating for all snacks, sweet-and-fat foods being specifically targeted. Intake of thylakoids also decreased subjective liking for sweet, scored directly following consumption.

The promotion of satiety by thylakoids found in this example is an entirely novel finding. This started 60 minutes after breakfast, suggesting enhanced early satiety signalling. In addition, ratings for satiety in the treated group were also higher several hours after lunch, compared to placebo. The enhanced satiety may be explained by increased secretion of satiety hormones CCK, both at an early time point and at later time points.

Treatment also had an attenuating effect on hunger ratings throughout the day. In comparison to ratings for satiety, the difference in hunger ratings reached significance later, at 180 minutes after breakfast in comparison to 60 minutes for satiety. The present findings on hunger verify previous findings on decreased hunger ratings following treatment with green-plant membranes. The suppressed hunger may be related to reduced ghrelin-levels and an indirect effect of increased circulating levels of the satiety hormones CCK and GLP-1. The later appearance of hunger suppression suggests an indirect effect by thylakoids on promotion of satiety hormones.

Besides increasing satiety and reducing hunger, in this study treatment with green-plant membranes also reduced wanting for all categories of palatable snacks; salty, sweet, and sweet-and-fat snacks as well as all snacks together, compared to control. The present results demonstrate that treatment with green-plant membranes suppress hedonic hunger directly.

Without being bond to any theory, the suppressed hedonic hunger by thylakoids may be an effect of altered levels of the reward-related appetite hormone GLP-1.

In the present study, correlation analyses demonstrated that individuals who scored high for emotional eating behaviour had greater effect of green-plant membranes on reducing wanting for palatable food, which is an entirely novel finding. Emotional eating is more common in women than men, and has been associated with cravings and increased consumption of sweet-and-fat foods, especially in response to stressors and negative emotions. Several studies have shown that the correlation between eating behaviour types and BMI is strongest for emotional eating behaviour, and higher in overweight subjects. Therefore, to affect overeating specifically in emotional eaters is very important.

Findings on reduced liking are important, since liking of sweetness influence wanting of sweet-tasting products. Indeed, overweight women have been shown to have an increased liking for sweet. A reduced liking may, over time, cause a reduction in wanting for sweet. As a consequence the reduced wanting may decrease snacking in between meals and thus overeating. In this study, treatment reduced wanting for sweet to a greater extent compared to other kinds of snacks. There was also an earlier onset. Wanting for sweet was reduced from 15 minutes and onwards, in comparison to wanting for salty and sweet-and-fat snacks, which were reduced from 60 minutes respectively. This implies that acute treatment with green-plant membranes in particular curbes the sweet taste. The mechanism for this targeting is not known.

The present example shows that treatment with green-plant membranes attenuate hunger, homeostatic and hedonic, as well as wanting for palatable food and liking for sweet. Reducing wanting is important, since wanting is a major cause of hedonic eating, which contributes to overconsumption and obesity. In addition, individuals who are obese and/or dieting are even more susceptible to hedonic hunger. Therefore, reducing cravings for palatable food is necessary, both to control appetite, prevent weight gain and to facilitate a permanent weight loss. Supplementation by green-plant membranes may help prevent eating between meals, hence over-eating and in the long run, overweight.

## Claims

1. Non-therapeutic use of isolated thylakoids to reduce the urge for palatable food, in order to decrease hedonic hunger, in man, wherein said use includes intake of isolated thylakoids.

2. The use according to claim 1, wherein the palatable food has one or several of the following characteristics:
- a glycemic index of at least 60, such as at least 70;
- high sugar content of at least 5 wt% for drinks and at least 20 wt%, such as at least 50 wt%, for other food;
- comprises a sweetener;
- a high fat content of at least 20 wt% and high content of sugar of at least 20 wt%;
- a high fat content of at least 20 wt% and a glycemic index of at least 50; and
- a high-fat content of at least 20 wt% and salt content (NaCl) of at least 1.0 wt%.

3. The use according to claim 2, wherein the palatable food has at least one of the following characteristics:
- high sugar content of at least 5 wt% for drinks and at least 20 wt%, such as at least 50 wt%, for other foodstuff; and
- a high fat content of at least 20% and high content of sugar of at least 20 wt%;
wherein the sugars comprised in the palatable food are selected from the group consisting of the monosaccharides glucose, fructose and galactose, the disaccharides sucrose, maltose and lactose, and mixtures thereof.

4. The use according to any one of the claims 1 to 3, wherein the release of GLP-1 (glucagon-like peptide-1) is increased by said use of isolated thylakoids.

5. The use according to any one of the claims 1 to 4, wherein the rewarding effect of palatable food, affecting the reward center in the brain, is decreased by said use of isolated thylakoids.

6. Non-therapeutic use of isolated thylakoids to decrease the rewarding effect of palatable food, such as palatable food according to any one of the claims 2 or 3

7. The use according to any one of the claims 1 to 6, wherein the isolated thylakoids have been isolated from green leaves of plants or from green algae; preferably the thylakoids, have been isolated from leaves of clover, rape, sugar beet, dandelion, Arabidopsis thaliana, maize, tobacco, sun flower, Chenopodium, Atriplex, spinach, mangold, quinoa, kale, or grasses; more preferred the thylakoids have been isolated from spinach.

8. The use according to any one of the claims 1 to 7, wherein the isolated thylakoids used are an integral part of a nutraceutical composition.

9. The use according to claim 8, wherein the content of isolated thylakoids in the nutraceutical composition corresponds to a chlorophyll content of from about 10 to about 50 mg chlorophyll per gram of the composition (dry weight);
preferably the carbohydrate content of the nutraceutical composition is from 5 to 50 g carbohydrates per 100 g of the nutraceutical composition (dry weight); and/or the protein content of the nutraceutical composition is from 10 to 60 g protein per 100 g of the nutraceutical composition (dry weight).

10. The use according to claim 8 or 9, wherein the nutraceutical composition comprises a physiologically tolerable oil-in-water emulsion comprising isolated thylakoids.

11. The use according to claim 10, wherein the nutraceutical composition comprises probiotic bacteria and optionally a prebiotic agent.

12. The use according to claim 5, wherein the rewarding effect is decreased by increasing the release of GLP-1 (glucagon-like peptide-1).

13. The use according to claim 1, wherein hedonic hunger is decreased by increasing the release of GLP-1 (glucagon-like peptide-1).

## Patentansprüche

1. Nichttherapeutische Verwendung von isolierten Thylakoiden, um den Drang nach wohlschmeckenden Lebensmitteln zu verringern, um hedonischen Hunger in einem Menschen zu vermindern, wobei diese Verwendung die Aufnahme isolierter Thylakoide umfasst.

2. Die Verwendung gemäß Anspruch 1, wobei das wohlschmeckende Lebensmittel eines oder mehrere der folgenden Charakteristika aufweist:
- einen glykämischen Index von mindestens 60, wie zum Beispiel mindestens 70;
- einen hohen Zuckergehalt von wenigstens 5 Gew.-% bei Getränken und wenigstens 20 Gew.-%, wie zum Beispiel wenigstens 50 Gew.-% bei anderen Nahrungsmitteln;
- enthaltend einen Süßstoff;
- einen hohen Fettgehalt von wenigstens 20 Gew.-% und einen hohen Zuckergehalt von wenigstens 20 Gew.-%;
- einen hohen Fettgehalt von wenigstens 20 Gew.-% und einen glykämischen Index von mindestens 50, und
- einen hohen Fettgehalt von wenigstens 20 Gew.-% und einen Salzgehalt (NaCl) von wenigstens 1,0 Gew.-%.

3. Die Verwendung gemäß Anspruch 2, wobei das wohlschmeckende Lebensmittel wenigstens eines der folgenden Charakteristika aufweist:
- einen hohen Zuckergehalt von wenigstens 5 Gew.-% bei Getränken und wenigstens 20 Gew.-%, wie zum Beispiel wenigstens 50 Gew.-% bei anderen Nahrungsmitteln; und
- einen hohen Fettgehalt von wenigstens 20 Gew.-% und einen hohen Zuckergehalt von wenigstens 20 Gew.-%,
wobei die Zucker, die in dem wohlschmeckenden Lebensmittel enthalten sind, ausgewählt sind aus der Gruppe, bestehend aus den Monosacchariden Glucose, Fructose und Galactose, den Disacchariden Sucrose, Maltose und Lactose und Mischungen daraus.

4. Die Verwendung gemäß einem der Ansprüche 1 bis 3, wobei die Freisetzung von GLP-1 (dem glucagonähnlichen Peptid 1) durch die Verwendung von isolierten Thylakoiden erhöht wird.

5. Die Verwendung gemäß einem der Ansprüche 1 bis 4, wobei der Belohnungseffekt von wohlschmeckenden Lebensmitteln, der das Belohnungszentrum im Gehirn beeinflusst, durch die Verwendung der isolierten Thylakoide vermindert wird.

6. Nichttherapeutische Verwendung von isolierten Thylakoiden, um den Belohnungseffekt wohlschmeckender Lebensmittel, wie der wohlschmeckenden Lebensmittel gemäß einem der Ansprüche 2 oder 3, zu vermindern.

7. Die Verwendung gemäß einem der Ansprüche 1 bis 6, wobei die isolierten Thylakoide aus grünen Blättern von Pflanzen oder aus grünen Algen isoliert wurden, wobei die Thylakoide bevorzugt aus Blättern von Klee, Raps, Zuckerrübe, Löwenzahn, Arabidopsis thaliana, Mais, Tabak, Sonnenblume, Chenopodium, Atriplex, Spinat, Mangold, Chinoa, Grünkohl oder Gräsern isoliert wurden, weiter bevorzugt wurden die Thylakoide aus Spinat isoliert.

8. Die Verwendung gemäß einem der Ansprüche 1 bis 7, wobei die verwendeten isolierten Thylakoide ein integraler Teil einer ernährungswirksamen Zusammensetzung sind.

9. Die Verwendung gemäß Anspruch 8, wobei der Gehalt isolierter Thylakoide in der ernährungswirksamen Zusammensetzung einem Chlorophyllgehalt von ungefähr 10 bis ungefähr 50 mg Chlorophyll pro Gramm der Zusammensetzung (als Trockengewicht) entspricht,
wobei der Kohlenhydratanteil der ernährungswirksamen Zusammensetzung von 5 bis 50 g Kohlenhydrate pro 100 g der ernährungswirksamen Zusammensetzung (als Trockengewicht) reicht; und/oder der Proteingehalt der ernährungswirksamen Zusammensetzung von 10 bis 60 g Protein pro 100 g der ernährungswirksamen Zusammensetzung (als Trockengewicht) reicht.

10. Die Verwendung gemäß Anspruch 8 oder 9, wobei die ernährungswirksame Zusammensetzung eine physiologisch tolerierbare Öl-in-Wasser-Emulsion, die die isolierten Thylakoide enthält, umfasst.

11. Die Verwendung gemäß Anspruch 10, wobei die ernährungswirksame Zusammensetzung probiotische Bakterien und gegebenenfalls ein probiotisches Agens enthält.

12. Die Verwendung gemäß Anspruch 5, wobei der Belohnungseffekt durch das Erhöhen der Freisetzung von GLP-1 (glucagonähnlichem Peptid 1) vermindert wird.

13. Die Verwendung gemäß Anspruch 1, wobei hedonischer Hunger durch das Erhöhen der Freisetzung von GLP-1 (glucagonähnlichem Peptid 1) vermindert wird.

## Revendications

1. Utilisation non thérapeutique de thylacoïdes isolés pour réduire l'envie pressante d'aliment appétissant, de manière à réduire la faim hédonique, chez l'homme, dans laquelle ladite utilisation inclut la prise de thylacoïdes isolés.

2. Utilisation selon la revendication 1, dans laquelle l'aliment appétissant présente une ou plusieurs des caractéristiques suivantes :
- un indice glycémique d'au moins 60, par exemple d'au moins 70 ;
- une teneur en sucre élevée d'au moins 5 % en poids pour les boissons et d'au moins 20 % en poids, par exemple d'au moins 50 % en poids, pour un autre aliment ;
- comprend un édulcorant ;
- une teneur en graisse élevée d'au moins 20 % en poids et une teneur en sucre élevée d'au moins 20 % en poids ;
- une teneur en graisse élevée d'au moins 20 % en poids et un indice glycémique d'au moins 50 ; et
- une teneur en graisse élevée d'au moins 20 % en poids et une teneur en sel (NaCl) d'au moins 1,0 % en poids.

3. Utilisation selon la revendication 2, dans laquelle l'aliment appétissant présente au moins l'une des caractéristiques suivantes :
- une teneur en sucre élevée d'au moins 5 % en poids pour les boissons et d'au moins 20 % en poids, par exemple d'au moins 50 % en poids, pour d'autres aliments ; et
- une teneur en graisse élevée d'au moins 20 % et une teneur en sucre élevée d'au moins 20 % en poids ;
dans laquelle les sucres compris dans l'aliment appétissant sont sélectionnés dans le groupe constitué des monosaccharides glucose, fructose et galactose, des disaccharides sucrose, maltose et lactose, et de mélange de ceux-ci.

4. Utilisation selon l'une quelconque des revendications 1 à 3, dans laquelle la libération du GLP-1 (peptide de type glucagon 1) est accrue par ladite utilisation de thylacoïdes isolés.

5. Utilisation selon l'une quelconque des revendications 1 à 4, dans laquelle l'effet de satisfaction de l'aliment appétissant, affectant le centre de satisfaction dans le cerveau, est réduit par ladite utilisation de thylacoïdes isolés.

6. Utilisation non thérapeutique de thylacoïdes isolés pour réduire l'effet de satisfaction d'un aliment appétissant, par exemple d'un aliment appétissant selon l'une quelconque des revendications 2 ou 3.

7. Utilisation selon l'une quelconque des revendications 1 à 6, dans laquelle les thylacoïdes ont été isolés de feuilles vertes de plantes ou d'algues vertes ; de préférence les thylacoïdes ont été isolés de feuilles de trèfle, navette, betterave à sucre, pissenlit, Arabidopsis thaliana, maïs, tabac, tournesol, Chenopodium, Atriplex, épinard, betterave champêtre, quinoa, chou frisé ou d'herbes ; de manière préférée entre tous les thylacoïdes ont été isolés de l'épinard.

8. Utilisation selon l'une quelconque des revendications 1 à 7, dans laquelle les thylacoïdes isolés font partie intégrante d'une composition nutraceutique.

9. Utilisation selon la revendication 8, dans laquelle la teneur en thylacoïdes isolés dans la composition nutraceutique correspond à une teneur en chlorophylle d'environ 10 à environ 50 mg de chlorophylle par gramme de la composition (poids sec) ;
de préférence la teneur en glucides de la composition nutraceutique est de 5 à 50 g de glucides pour 100 g de la composition nutraceutique (poids sec) ; et/ou
la teneur en protéine de la composition nutraceutique est de 10 à 60 g de protéine pour 100 g de la composition nutraceutique (poids sec).

10. Utilisation selon la revendication 8 ou 9, dans laquelle la composition nutraceutique comprend une émulsion huile-dans-l'eau physiologiquement tolérable comprenant des thylacoïdes isolés.

11. Utilisation selon la revendication 10, dans laquelle la composition nutraceutique comprend des bactéries probiotiques et facultativement un agent prébiotique.

12. Utilisation selon la revendication 5, dans laquelle l'effet de satisfaction est réduit par augmentation de la libération de GLP-1 (peptide de type glucagon 1).

13. Utilisation selon la revendication 1, dans laquelle la faim hédonique est réduite par augmentation de la libération de GLP-1 (peptide de type glucagon 1).
